# EUROPEAN PATENT APPLICATION

(11) **EP 4 335 440 A1**
(43) Date of publication of application: **13.03.2024**
(21) Application number: 22798627.0
(22) Date of filing: 04.05.2022
(51) Int. Cl.: A61K 31/501, A61K 31/53, A61K 31/4439, A61K 31/444, C07D 403/12, A61P 35/00, A61P 37/00, A61P 29/00, A61P 19/02, A61P 17/00, A61P 17/06, A61P 1/00

(54) **NITROGEN-CONTAINING HETEROCYCLIC PYRIDINE COMPOUND**

(30) Priority: 04.05.2021 CN 202110487046; 07.08.2021 CN 202110904652; 08.09.2021 CN 202111049227
(71) Applicant: Shanghai Zheye Biotechnology Co., Ltd., Shanghai 201800 (CN)
(72) Inventor: ZHANG, Qiang, Shanghai 201800 (CN); DU, Fengtian, Shanghai 201800 (CN); YANG, Xiangyu, Shanghai 201800 (CN)
(74) Representative: Simmons & Simmons LLP (Munich)
(86) International application number: PCT/CN2022/090847
(87) International publication number: WO 2022/233286

(57) **Abstract**

A nitrogen-containing heterocyclic protein inhibitor compound, and a stereoisomer, a tautomer or a pharmaceutically acceptable salt thereof. Also provided are a preparation method for the nitrogen-containing heterocyclic compound, and a use thereof. A drug prepared from the compound is widely used for treating diseases, the diseases comprising multiple types of autoimmune diseases, such as multiple sclerosis, rheumatoid arthritis, inflammatory bowel disease, lupus erythematosus, neurodermatitis, dermatitis, psoriasis, psoriatic arthritis, Crohn's disease, dry syndrome, and scleroderma.

## Description

### TECHNICAL FIELD

The present invention relates to a nitrogen-containing heterocyclic compound, which can regulate the signal transduction of multiple cytokines by inhibiting TYK2. The present invention also relates to a method for preparing the compound and use thereof in treating diseases.

### BACKGROUND ART

Nitrogen-containing heterocycles are a class of nitrogen-containing heterocyclic compounds with special structures and extensive biological activities. Since the successful development of nitrogen-containing heterocyclic herbicides, research on nitrogen-containing heterocyclic compounds has rapidly developed. For example, nitrogen-containing heterocyclic mycotoxin is the first nitrogen-containing heterocyclic compound with natural bactericidal activity. Research has found that nitrogen-containing heterocyclic compounds have good biological activities in pesticides and pharmaceuticals, such as weeding, disinsection, antibiosis, antivirus and antihypertension.

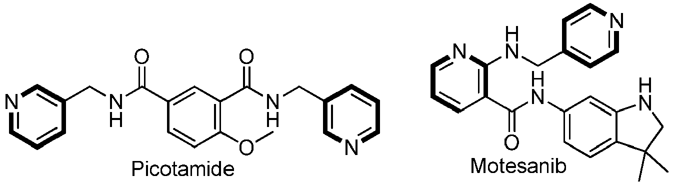

The cytokines IL-12 and IL-23 activate antigen presenting cells and play an important role in the differentiation and proliferation of T cells. These cytokines are involved in mediating various autoimmune diseases, including rheumatoid arthritis, multiple sclerosis, inflammatory bowel disease and lupus erythematosus.

Tyrosine kinase 2 (TYK2) is a member of the JAK family (other members include JAK1, JAK2 and JAK3) and is responsible for participating in the signal transduction of IFN-α, IL-6, IL-10 and IL-12. TYK2 can promote phosphorylation of STAT proteins downstream of IL-12, IL-23 and type I interferon receptor. By inhibiting the activity of TYK2, various autoimmune diseases, including multiple sclerosis, rheumatoid arthritis, inflammatory bowel disease, lupus erythematosus, neurodermatitis, dermatitis, psoriasis, psoriatic arthritis, Crohn's disease, Sjogren's syndrome and scleroderma, can be effectively treated.

How to design TYK2 inhibitors with better efficacy, pharmacokinetic properties and superior drug-likeness is a huge challenge faced by pharmaceutical chemists. In recent years, although a series of TYK2 inhibitors have been disclosed, there is still a need to discover and develop new compounds with better efficacy, pharmacokinetic properties, and better physicochemical characteristics. The present invention designed the compounds with the structures of a general formula (I), and found that such compounds exhibit excellent TYK2 inhibitory effect and comprehensive performance.

### SUMMARY OF THE INVENTION

The present invention provides a compound of formula (I): 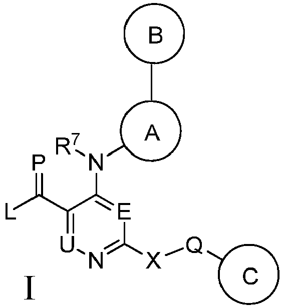 or a stereoisomer, a tautomer or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
ring B is aryl, heteroaryl, or heterocyclyl, and ring B is selected from: 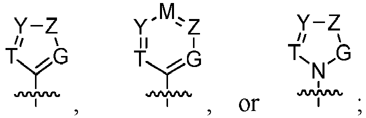
wherein, T, G, Y, Z, and M are each independently selected from oxygen atom, CR^{A1}, CR^{A2}, nitrogen atom or NR^{B};
E is nitrogen atom or carbon atom;
R^{A1} and R^{A2} are selected from the group consisting of hydrogen, deuterium, C₁₋₆ alkyl, halogen, 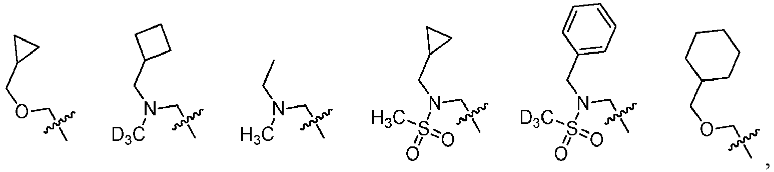 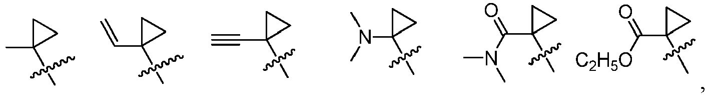 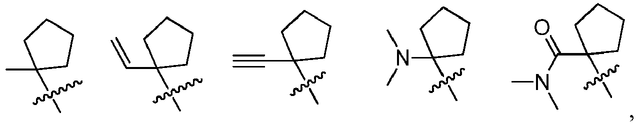 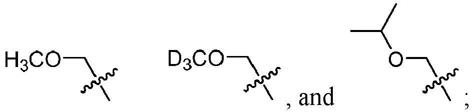
R^{B} is selected from the group consisting of hydrogen, C₁₋₆ alkyl, C₁₋₆ deuterated alkyl, C₁₋₆ alkyl-C(O)-, C₁₋₆ haloalkyl-C(O)-, cycloalkyl-C(O)-, aryl-C(O)-, substituted amino-C(O)-, C₁₋₆ alkyl-S(O)₂-, alkenyl, deuterated alkenyl, alkynyl, deuterated alkynyl, 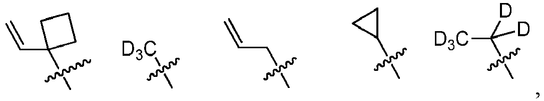 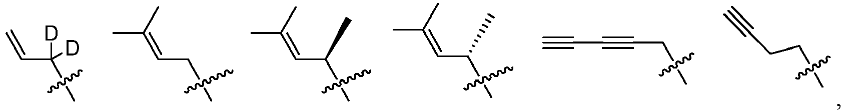 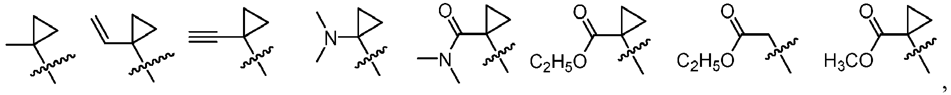 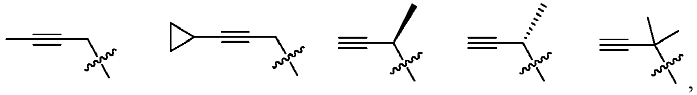 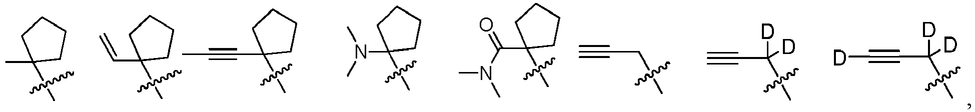 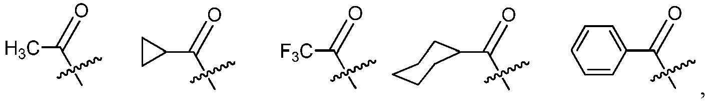 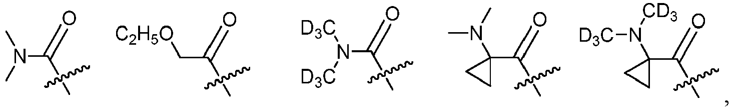 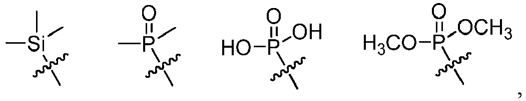 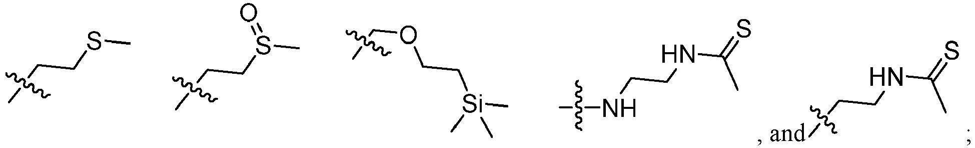
Q is a chemical bond, -C(O)-, -C(S)-, -S(O)-, -S(O)₂-, or -C(N-R⁸)-, wherein -C(O)-, -C(S)-, -S(O)-, -S(O)₂-, and -C(N-R⁸)- are: 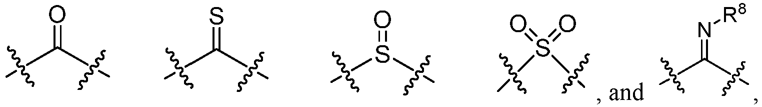 respectively;
   P is oxygen atom or sulfur atom;
   X is chemical bond, oxygen atom, NH or NR^{A};
   R^{A} is alkyl, deuterated alkyl, or haloalkyl;
   U is nitrogen atom or carbon atom;
   ring A is aryl, heteroaryl, or heterocyclyl, and ring A is selected from the group consisting of: 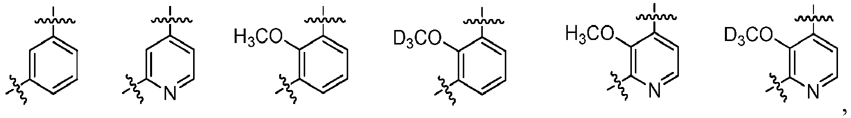 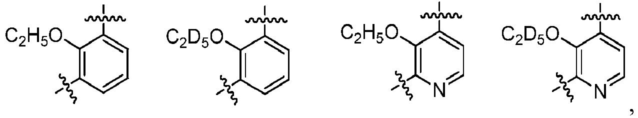 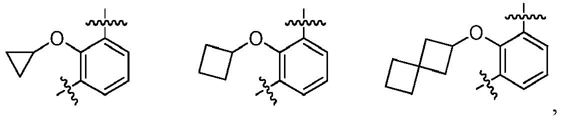 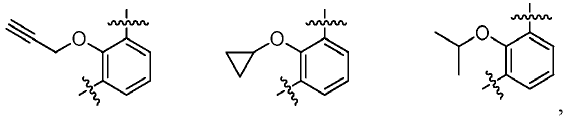 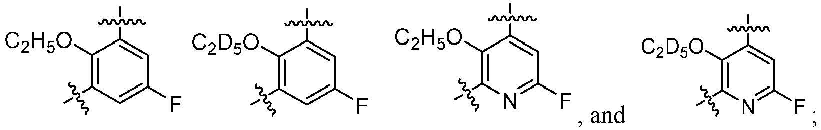
   C is alkyl, cycloalkyl, amino, substituted amino, aryl, heteroaryl, or heterocyclyl, and C is selected from the group consisting of: 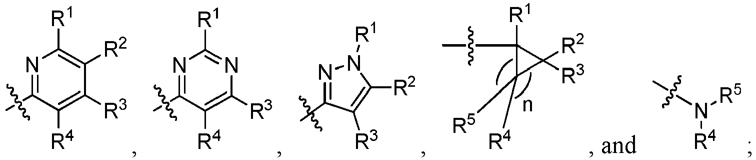
   wherein, R¹, R², R³, R⁴, R⁵, R⁷, and R⁸ are selected from the group consisting of hydrogen, deuterium, halogen, amino, alkynyl, deuterated alkynyl, alkenyl, deuterated alkenyl, alkenylcarbonyl, deuterated alkenylcarbonyl, alkyl, deuterated alkyl, alkylcarbonyl, and deuterated alkylcarbonyl; wherein, the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
   n is 1, 2, 3, 4, 5, or 6.

The present invention provides a compound selected from the group consisting of: 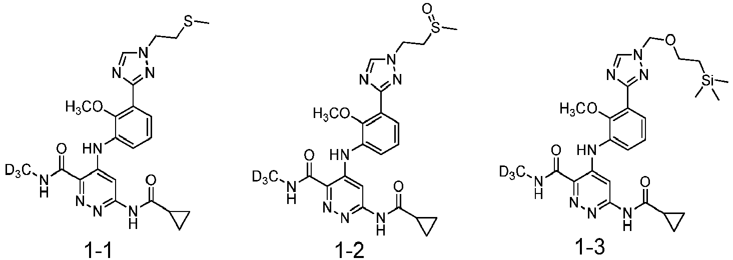 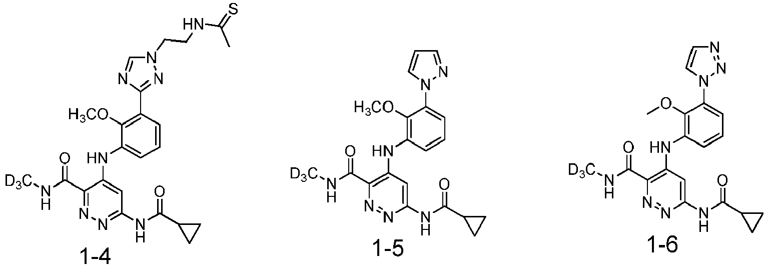 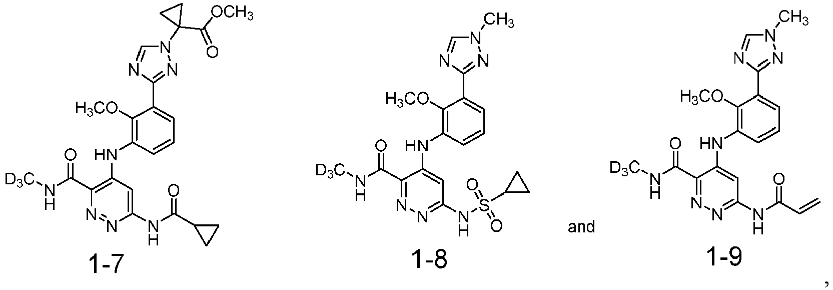 or a stereoisomer, a tautomer, or a pharmaceutically acceptable salt thereof.

The present invention provides a compound of formula (I-1): 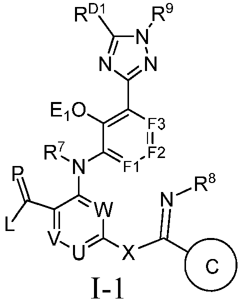 or a stereoisomer, a tautomer, or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
P is oxygen atom or sulfur atom;
X is oxygen atom, NH, or NR⁹;
V, U, and W are nitrogen or CR⁶;
R^{D1} is selected from hydrogen, C₁₋₆ alkyl, or halogen;
F1, F2, and F3 are nitrogen atom or carbon atom;
E₁ is hydrogen, deuterium, alkyl, or deuterated alkyl;
C is alkyl, cycloalkyl, amino, substituted amino, aryl, heteroaryl, or heterocyclyl, and ring C is selected from: 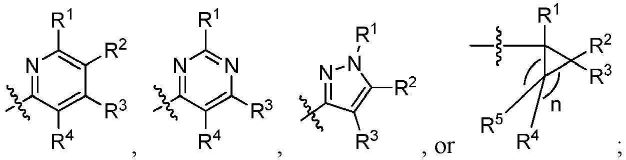
wherein, R¹, R², R³, R⁴, R⁵, R⁶, R⁷, and R⁸ are selected from the group consisting of hydrogen, deuterium, halogen, amino, alkynyl, alkenyl, deuterated alkenyl, alkyl, and deuterated alkyl; wherein the alkynyl, alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

The present invention provides a compound selected from the group consisting of: 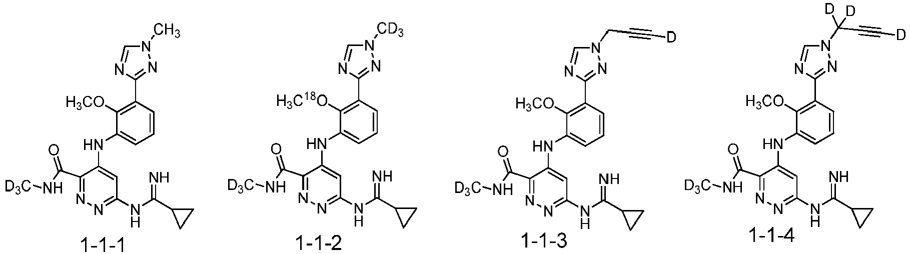 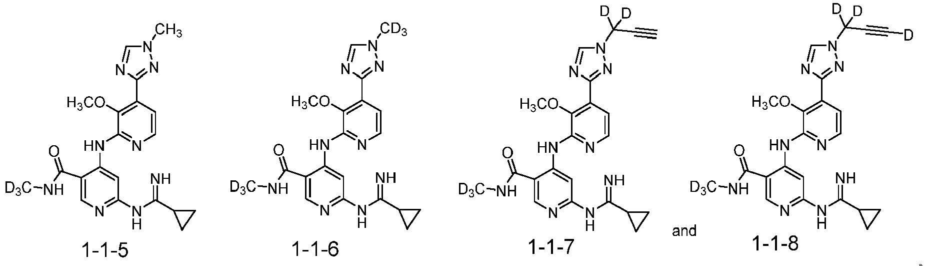 or a stereoisomer, a tautomer, or a pharmaceutically acceptable salt thereof.

The present invention provides a compound of formula (II-1): 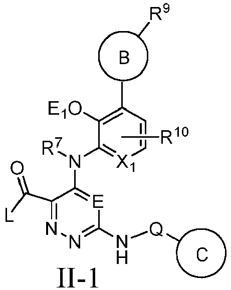 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
Q is a chemical bond, -C(O)-, -C(S)-, -S(O)-, or -S(O)₂-, wherein -C(O)-, -C(S)-, -S(O)-, and -S(O)₂- are: 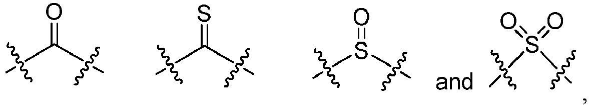 respectively;
C is alkyl, cycloalkyl, amino, substituted amino, aryl, heteroaryl, or heterocyclyl, and C is selected from the group consisting of: 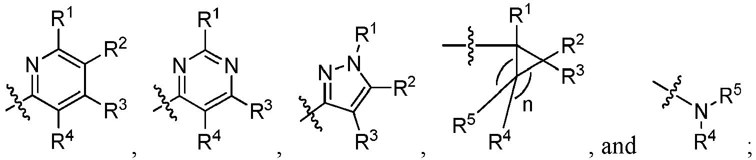
wherein, R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

The present invention provides a compound of formula (II-1A): 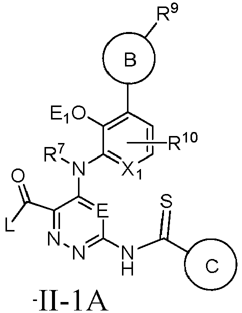 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
C is alkyl, cycloalkyl, amino, substituted amino, aryl, heteroaryl, or heterocyclyl, and C is selected from the group consisting of: 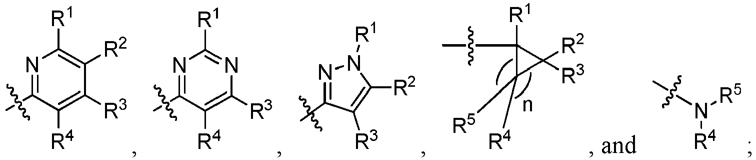
wherein, R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

The present invention provides a compound selected from the group consisting of: 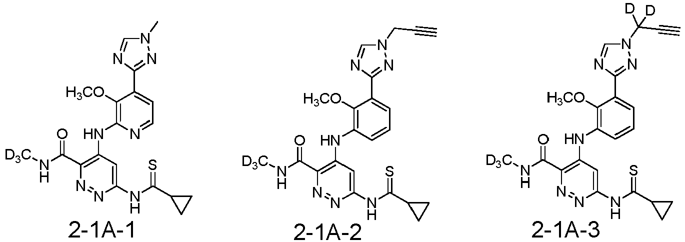 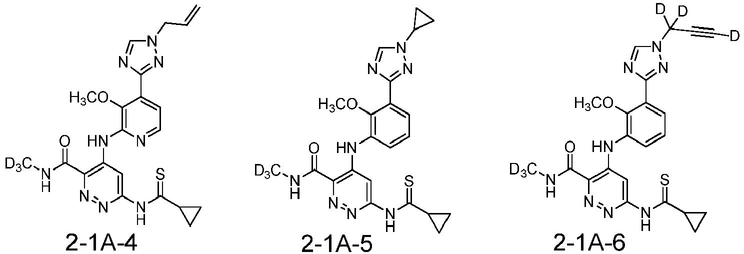 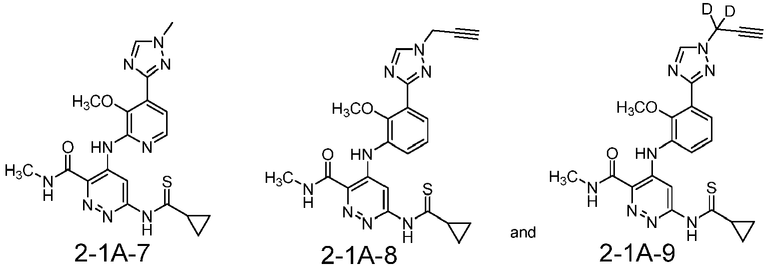 or a stereoisomer, a tautomer, or a pharmaceutically acceptable salt thereof.

The present invention provides a compound of formula (II-1B): 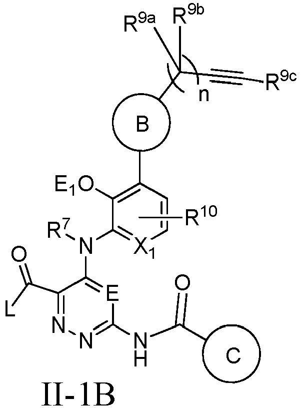 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
C is alkyl, cycloalkyl, amino, substituted amino, aryl, heteroaryl, or heterocyclyl, and C is selected from the group consisting of: 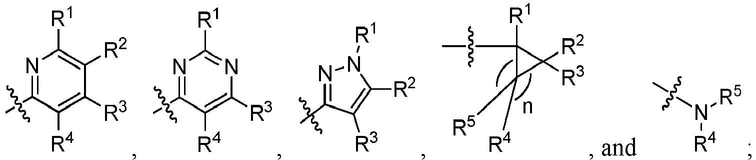
wherein, R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from the group consisting of hydrogen, deuterium, alkyl, deuterated alkyl, haloalkyl, cycloalkyl, deuterated cycloalkyl, alkynyl, and deuterated alkynyl, or R^{9a} and R^{9b} together with the carbon atom(s) to which they are attached form a cycloalkyl;
n is 1, 2, or 3.

The present invention provides a compound selected from the group consisting of: 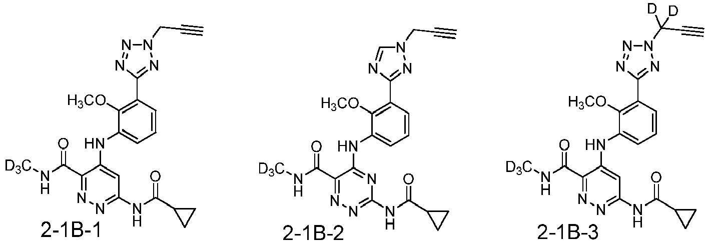 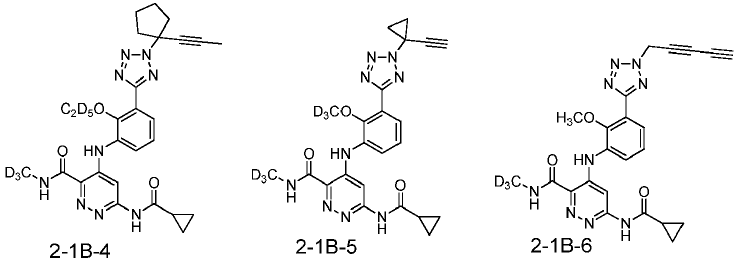 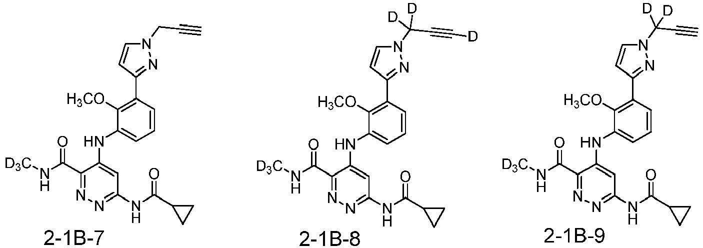 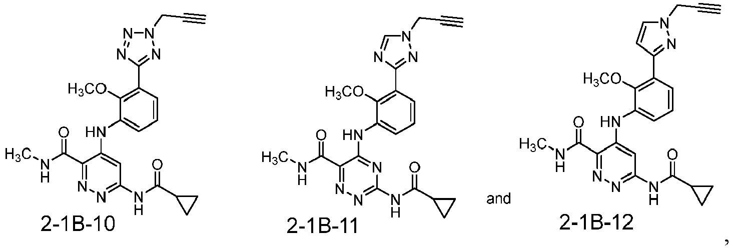 or a stereoisomer, a tautomer, or a pharmaceutically acceptable salt thereof.

The present invention provides a compound of formula (II-2): 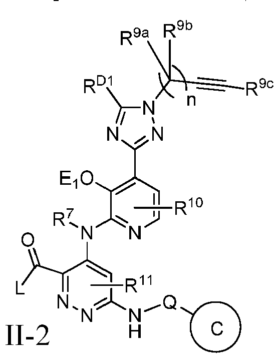 or a stereoisomer, a tautomer, or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
Q is a chemical bond or carbonyl;
C is alkyl, cycloalkyl, amino, substituted amino, aryl, heteroaryl, or heterocyclyl, and C is selected from the group consisting of: 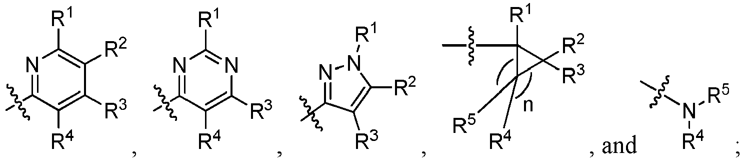
wherein, R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from the group consisting of hydrogen, deuterium, alkyl, deuterated alkyl, haloalkyl, cycloalkyl, deuterated cycloalkyl, alkynyl, and deuterated alkynyl, or R^{9a} and R^{9b} together with the carbon atom(s) to which they are attached form a cycloalkyl;
n is 1, 2, or 3.

The present invention provides a compound of formula (II-2-1): 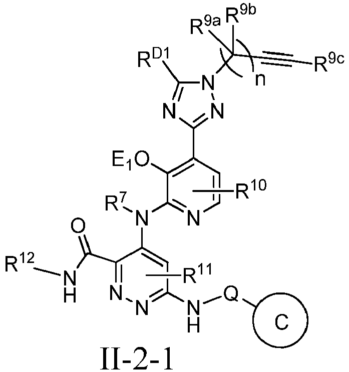 or a stereoisomer, a tautomer, or a pharmaceutically acceptable salt thereof,
wherein, R¹² is deuterium, hydrogen, alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
Q is chemical bond or carbonyl;
C is alkyl, cycloalkyl, amino, substituted amino, aryl, heteroaryl, or heterocyclyl, and C is selected from the group consisting of: 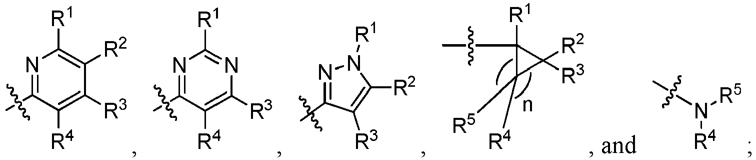
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from hydrogen, deuterium, alkyl, deuterated alkyl, haloalkyl, cycloalkyl, deuterated cycloalkyl, alkynyl, or deuterated alkynyl, or R^{9a} and R^{9b} together with the carbon atom(s) to which they are attached form a cycloalkyl;
n is 1, 2, or 3.

The present invention provides a compound of formula (II-2-2): 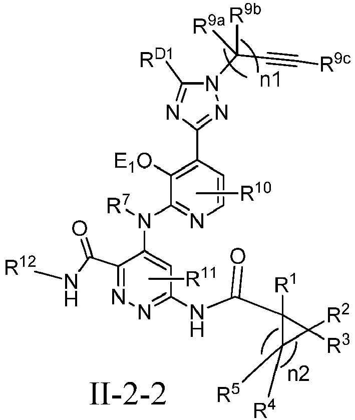 or a stereoisomer, a tautomer, or a pharmaceutically acceptable salt thereof,
wherein, R¹² is deuterium, hydrogen, alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
wherein, R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from hydrogen, deuterium, alkyl, deuterated alkyl, haloalkyl, cycloalkyl, deuterated cycloalkyl, alkynyl, or deuterated alkynyl, or R^{9a} and R^{9b} together with the carbon atom(s) to which they are attached form a cycloalkyl;
n1 is 1, 2, or 3;
n2 is 1, 2, or 3.

The present invention provides a compound selected from the group consisting of: 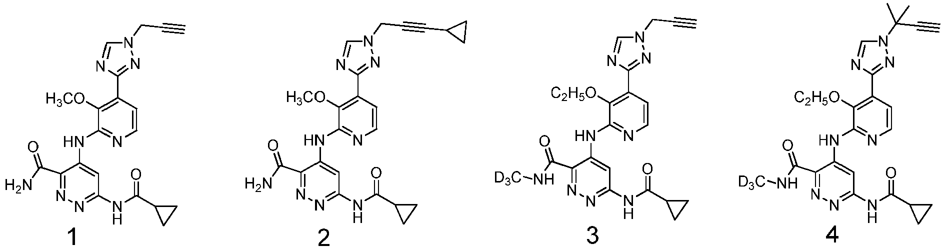 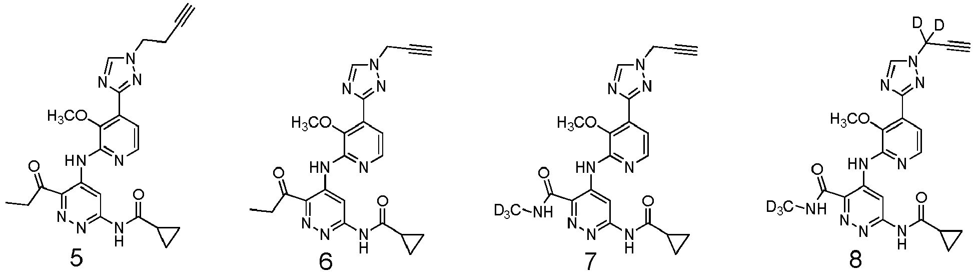 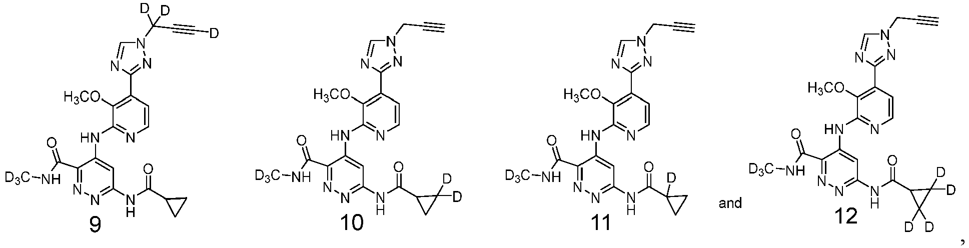 or a stereoisomer, a tautomer, or a pharmaceutically acceptable salt thereof.

The present invention provides a pharmaceutical composition, comprising one or more of the compounds according to any item of the present invention and a pharmaceutically acceptable carrier or diluent.

Further provided is a use of the compound according to any item of the present invention in the manufacture of a medicament for treating a disease, wherein the disease is an inflammatory or autoimmune disease or a cancer mediated by kinase TYK2, including multiple sclerosis, rheumatoid arthritis, inflammatory bowel disease, lupus erythematosus, neurodermatitis, dermatitis, atopic dermatitis, psoriasis, psoriatic arthritis, Crohn's disease, Sjogren's syndrome, or scleroderma.

### DETAILED DESCRIPTION OF THE INVENTION

All technical and scientific terms used in this description have the meaning commonly understood by those ordinary skilled in the art.

The term "hydrogen" as used herein refers to -H.

The term "deuterium" as used herein refers to -D.

The term "halogen" as used herein refers to -F, -Cl, -Br and -I.

The term "oxygen atom" as used herein refers to O.

The term "carbon atom" as used herein refers to C.

The term "nitrogen atom" as used herein refers to N.

The term "sulfur atom" as used herein refers to S.

The term "carbonyl" as used herein refers to -C(O)-.

The term "amino" as used herein refers to -NH₂.

The term "hydroxyl" as used herein refers to -OH.

The term "alkyl" as used herein refers to a saturated aliphatic hydrocarbyl group having from 1 to 10 carbon atoms, and includes both straight chain and branched chain hydrocarbon groups. Non-limiting examples of alkyl groups include methyl, ethyl, n-propyl, isopropyl, n-butyl, isobutyl, sec-butyl, tert-butyl, n-pentyl, neopentyl, n-hexyl, and the like. The alkyl group described herein can be optionally substituted with one or more substituents selected from the group consisting of fluorine, chlorine, bromine, iodine, cyano, nitro, hydroxyl, carboxyl, amino, alkyl, alkoxyl, acyl, acyloxy, oxo, amido, ester, amine, cycloalkyl, cycloalkenyl, heterocycloalkyl, alkenyl, alkenyloxy, alkynyl, cycloalkoxy, heterocycloalkyloxy, aryloxy, heteroaryloxy, aryl, and heteroaryl.

The term "cycloalkyl" refers to an alkyl group substituted by saturated or partially unsaturated monocyclic or polycyclic cyclic hydrocarbons. A cycloalkyl ring contains 3 to 20 carbon atoms, preferably 3 to 12 carbon atoms, and more preferably 3 to 10 carbon atoms. Non-limiting examples of monocyclic cycloalkyls include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyls include spiro-, fused- or bridged-ring cycloalkyl .

The term "aryl" as used herein refers to a 6- to 10-membered all-carbon monocyclic or fused polycyclic (i.e., a ring that shares a pair of adjacent carbon atoms) group, and polycyclic (i.e., a ring that has a pair of adjacent carbon atoms) group having conjugated π-electron system. The aryl group can be covalently attached to a defined chemical structure at any carbon atom that results in a stable structure. The aryl groups described herein can be optionally substituted with one or more substituents selected from the group consisting of fluorine, chlorine, bromine, iodine, cyano, nitro, hydroxyl, carboxyl, amino, alkyl, alkoxyl, acyl, amido, ester, amine, sulfonyl, sulfinyl, cycloalkyl, cycloalkenyl, heterocycloalkyl, alkenyl, alkynyl, and cycloalkoxyl.

The term "heteroaryl" as used herein refers to an aromatic group consisting of 5 to 10 atoms and containing at least one heteroatom selected from N, O, or S. A heteroaryl may have a single ring (non-limiting examples include furan, thiophene, imidazole, pyrazole, pyridine, pyrazine, oxazole, thiazole, and the like.), or multiple fused rings (non-limiting examples include benzothiophene, benzofuran, indole, isoindole, and the like.), wherein the fused ring may or may not be an aromatic group containing a heteroatom, assuming that the point of attachment is via an atom of the aromatic heteroaryl group. The heteroaryl groups described herein can be optionally substituted with one or more substituents selected from the group consisting of fluorine, chlorine, bromine, iodine, cyano, nitro, hydroxyl, amino, alkyl, alkoxyl, acyl, acyloxy, amido, ester, amine, sulfonyl, sulfinyl, cycloalkyl, cycloalkenyl, heterocycloalkyl, alkenyl, alkynyl, and cycloalkoxyl.

The term "alkenyl" as used herein refers to an alkenyl group with 2 to 8 carbon atoms and at least one alkenyl unsaturated site. Non-limiting examples of alkenyl groups include vinyl, propenyl, allyl, isopropenyl, butenyl, isobutenyl, and the like. The alkenyl groups described herein can be optionally substituted with one or more substituents selected from the group consisting of deuterium, fluorine, chlorine, bromine, iodine, cyano, nitro, hydroxyl, carboxyl, amino, alkyl, alkoxyl, acyl, amido, ester, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, amino, silyl, phosphonyl, deuterated alkyl, heterocycloalkyl, aryl, heteroaryl, alkynyl, alkenyl, arylalkyl, and ester.

The term "alkynyl" as used herein refers to an alkyl group that involves two adjacent carbon atoms connected by a triple bond, wherein said alkyl group is as defined herein. Alkynyl refers to an unsaturated alkyl group as defined herein containing at least two carbon atoms and at least one carbon-carbon triple bond, such as ethynyl, 1-propinyl, 2-propinyl, 1-, 2- or 3-butynyl, and the like. The alkynyl group can be substituted or unsubstituted, and when substituted, the substituent(s) can be preferably one or more groups independently selected from the group consisting of deuterium, fluorine, chlorine, bromine, iodine, cyano, nitro, hydroxyl, carboxyl, amino, alkyl, alkoxyl, acyl, amido, ester, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, amino, silyl, phosphonyl, deuterated alkyl, heterocycloalkyl, aryl, heteroaryl, alkynyl, alkenyl, arylalkyl, and ester.

### EXAMPLES

The following examples are used to further illustrate the present invention, but the present invention is not limited to them. Throughout the present application, various examples of the compounds and methods of the present invention are referred to herein. The present invention is not limited to these examples, and the following examples are merely intended to provide methods for practicing the present invention and are not intended to limit the scope of the present invention by any means.

The compounds provided by the present invention can be prepared by standard synthetic methods well known in the art, and the present description provides general methods for preparing the compounds of the present invention. Starting materials are usually available commercially or can be prepared by methods well known to those skilled in the art.

### Process 1:

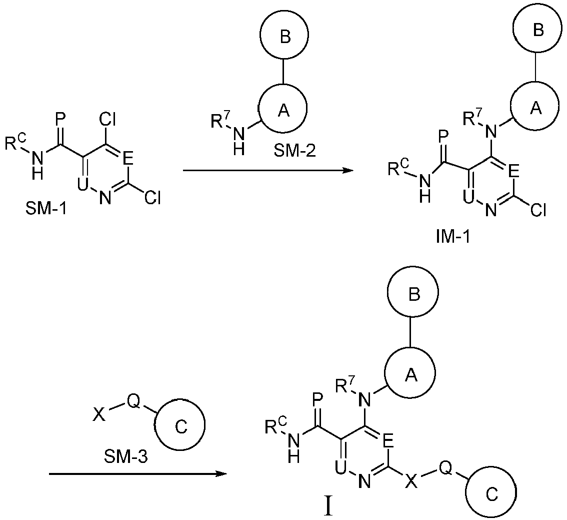

Firstly, SM-1 is used as starting material and is reacted with SM2 to obtain IM-1. Then IM-1 is reacted with SM-3 to give Compound I.

### Process 2:

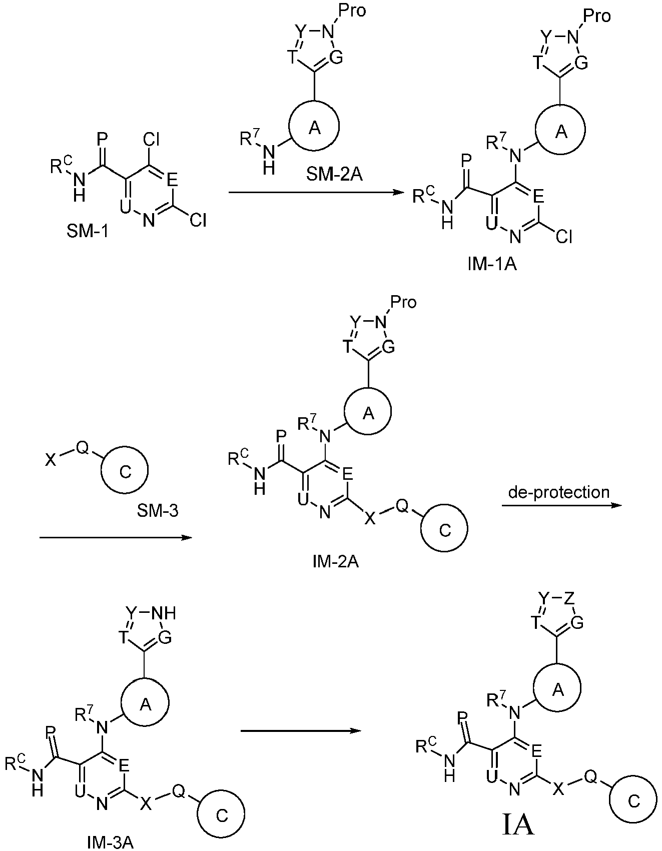

Firstly, SM-1 is used as starting material and is reacted with SM-2A to obtain IM-1A. Then IM-1A is reacted with SM-3 to obtain IM-2A, which is subjected to de-protection to give IM-3A. IM-3A is further reacted to give Compound IA.

The compounds of the present invention and the corresponding preparation methods are further explained and enumerated below by way of examples and preparations. It should be understood that although typical or preferred reaction conditions are provided in the specific examples, other reaction conditions may be used by those skilled in the art. The optimal reaction conditions may vary with the used particular reaction substrates or solvents, but the conditions may be determined through common optimization by those skilled in the art.

### Preparation of intermediates

Using 3-bromopropyne as the raw material, deuterated propargyl bromide was prepared by referring to the preparation scheme in the literature (Journal of Medicinal Chemistry (2004), 47 (2), 400-410). MS: m/z 262.1, [M+H]⁺.

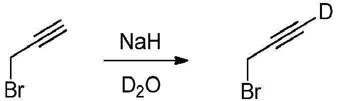

Deuterated propargyl bromide was prepared by referring to the preparation scheme in the literature (Journal of the Chinese Chemical Society (Taipei) (1998), 45(2), 307-312) and by using the corresponding deuterium reagent (deuterated water).

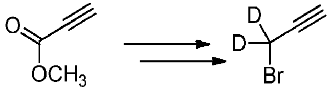

Deuterated propargyl bromide was prepared by referring to the preparation scheme in the literature (Bioorganic & Medicinal Chemistry (2013), 21(21), 6634-6641) and by using the corresponding deuterium reagent (deuterated lithium aluminum hydride).

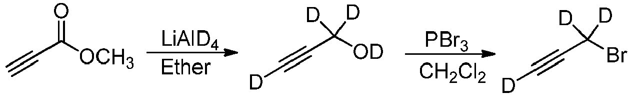

### Step 1

2.4 g of deuterated lithium aluminum hydride was dispersed in 150 ml of ether, cooled down to -50°C and slowly dropwise added with a solution of 6.0 g of methyl propiolate in 150 ml of ether. After the addition, the stirring was continued at -30°C, then the mixture was warmed up to room temperature and stirred overnight. The reaction solution was added with 3 ml of heavy water, sodium hydroxide (0.22 g dissolved in 1.5 ml of heavy water), and 2 ml of heavy water in sequence, and filtered. The filter cake was washed twice with 30 ml of ether. The filtrate was collected and concentrated under reduced pressure to obtain a dark yellow oily product, which was distilled under reduced pressure at 130°C to give 2.3 g of colorless oily substance.

### Step 2

1.2 g of deuterated propargyl alcohol was dissolved in 15 ml of dichloromethane, the obtained solution was cooled down to -5°C under nitrogen gas protection and slowly added with 6.0 g of phosphorus tribromide. After the addition, the stirring was continued at -5°C for 1 hour and then at room temperature. The reaction solution was added with 15 ml of ice water and then separated. The organic layer was washed with 25 ml of saturated solution of sodium bicarbonate and 25 ml of water successively, dried over anhydrous sodium sulfate, and concentrated under reduced pressure to remove the solvent. 1.6 g of a light-yellow oily liquid was obtained.

Alternatively, the following method was used for preparation:

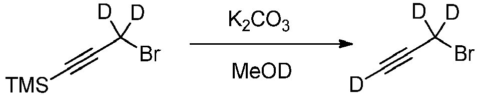

(3-bromoprop-1-yne-1-yl-3,3-*d2*)trimethylsilane (300 mg, 1.55 mmol), potassium carbonate (644 mg, 4.7 mmol), and methanol-OD (3 ml) were added into a 50 ml single-necked flask, and stirred and reacted at room temperature for 30 min after the addition. The reaction solution was filtered to remove the insoluble substances, and the filtrate was added with 50 ml of heavy water and extracted with ether. The organic phases were combined, dried over anhydrous sodium sulfate, filtered, and concentrated to give the product.

The following deuterated intermediates were prepared by referring to the preparation schemes in the literatures (WO2017181918; Journal of the American Chemical Society (1990), 112(8), 3156-3162; Journal of Organic Chemistry (1988), 53(20), 4748-4758; Organic Letters (2007), 9(16), 2981-2984; Bulletin of the Chemical Society of Japan (2003), 76(2), 347-353; Angewandte Chemie, International Edition (2016), 55(9), 3171-3175):

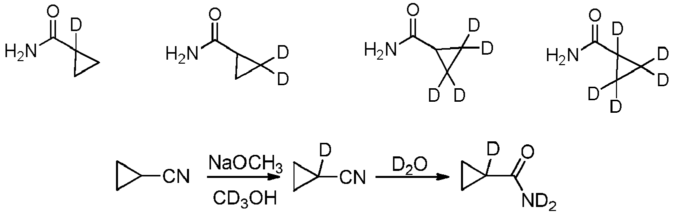

Cyanocyclopropane was added dropwise into a deuterated methanol solution containing sodium methoxide. The mixture was refluxed for 16 h, and concentrated under reduced pressure to remove deuterated methanol. A yellow solution (GC-MS: 69.1) was obtained after the concentration, added with a mixed solution of deuterated methanol and deuterated water, and refluxed for another 8 h. The mixture was concentrated under reduced pressure to remove the solvent and obtain the target product.

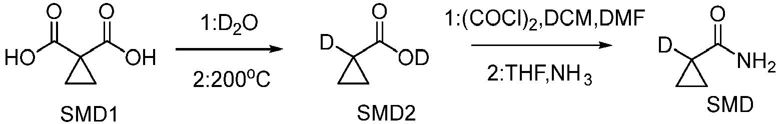

SMD1 (50 g) and heavy water (35 ml) were added into a 500 ml three-necked flask under nitrogen gas protection, heated to 100°C for dissolution and distilled under reduced pressure to remove most of the water (approximately 25 ml to 30 ml). The system was supplemented with heavy water (25 ml) and distilled again under reduced pressure to remove the water. By repeating such operations three times, hydrogen in the carboxyl was fully exchanged with deuterium in the heavy water. The system was heated to 160°C and distilled under reduced pressure to remove the water. The system was heated to 200°C and distilled under reduced pressure to collect the fraction of 180°C to 200°C. The distillation was completed in 5 to 10 minutes, and 22 g of a colorless oily fraction was obtained.

SMD2 (6.5 g), dichloromethane (30 ml), and DMF (0.5 ml) were added into a 250 ml three-necked flask under nitrogen gas protection, cooled down to 0 to -10°C, and dropwise added with oxalyl chloride (9.5 g) while maintaining an internal temperature below 0°C. After the addition, the mixture was kept at 0°C and reacted for 2-3 h until the reaction was detected to be completed by TLC. Dichloromethane was removed by concentration under reduced pressure. In another reaction flask, 150 ml of THF was added under nitrogen gas protection and cooled down to 0°C, then ammonia gas was introduced until it was saturated. The acyl chloride concentrated solution was added into the THF solution in batches, warmed up to 20°C after the addition and stirred for 20 minutes. The resulting solution was filtered, and the filtrate was concentrated to give 1.6 g of the target product.

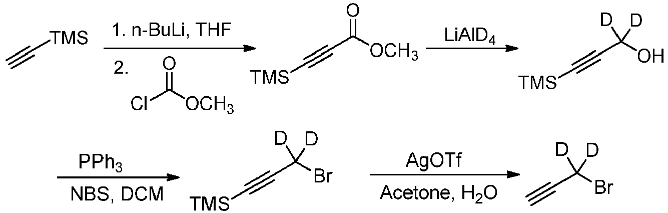

### Step 1

Trimethylsilylacetylene (10.0 g) and dried tetrahydrofuran (100 ml) were added into a 250 ml three-necked flask, cooled down to -80°C, and dropwise added with n-butyl lithium (40 ml). After the addition, the mixture was reacted for 30 minutes at -80°C, and then dropwise added with methyl chloroformate (10.6 g). After the addition, the resulting mixture was naturally warmed up to about -50°C and reacted for 30 minutes at the same temperature. The reaction was quenched by pouring the reaction solution into 200 ml of aqueous solution of ammonium chloride, and the obtained solution was extracted with 200 ml of ether. The organic layer was separated, dried and concentrated to give a crude product, which was then purified by silica gel column chromatography to obtain 8.0 g of a light yellow liquid. ¹H NMR (400 MHz, CDCl₃): δ 3.79 (s, 3H), 0.26 (s, 9H).

### Step 2

The product from the previous step (7.0 g) and ether (300 ml) were added into a 500 ml single-necked flask, cooled down to 0°C, and added with LiAlD₄ (1.5 g) in batches. After the addition, the mixture was reacted for 1 h at the same temperature. The reaction was quenched by slowly dropwise adding an appropriate amount of water into the reaction solution. After the addition, the resulting solution was added with an appropriate amount of anhydrous sodium sulfate for drying and filtered. The filtrate was concentrated to give a crude product, which was then purified by column chromatography to obtain 4.0 g of a colorless liquid. ¹H NMR (400 MHz, CDCl₃): δ 1.95 (br s, 1H), 0.18 (s, 9H).

### Step 3

The product from the previous step (4.0 g), triphenylphosphine (8.1 g), and dichloromethane (100 ml) were added into a 250 ml single-necked flask, cooled down to 0°C, and added with NBS (5.5 g, 30.9 mmol) in batches. After the addition, the mixture was stirred for 30 minutes. The resulting solution was concentrated under reduced pressure at 0°C to remove most of the dichloromethane solvent, and the residue was added with 100 ml of n-hexane, stirred and filtered. The filtrate was directly column chromatographed, and the eluent was concentrated under reduced pressure at 10°C to obtain approximately 3.0 g of a colorless liquid.

### Step 4

The product from the previous step (2.0 g), acetone (40 ml), water (1 ml) and silver trifluoromethanesulfonate (270 mg) were added into a 50 ml single-necked flask. After the addition, the mixture was stirred for 1 h at room temperature. The reaction solution was poured into 200 ml of saturated aqueous solution of ammonium chloride, and the obtained solution was extracted with 200 ml of ether. The organic layer was separated, dried and concentrated under reduced pressure at 0°C to give 2.0 g of a colorless liquid (containing a small amount of solvent), which was used directly.

### Preparation of compounds

### Example 1:

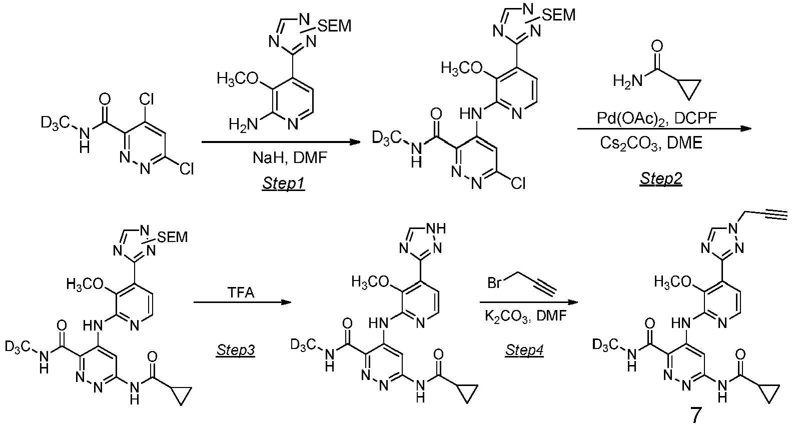

### Step 1

2-aminopyridine compound (3.0 g) and N,N-dimethylformamide (50 ml) were added into a reaction flask, cooled down to 0°C, and added with sodium hydride (1.5 g) in batches. After the addition, the mixture was stirred for 20 minutes, then slowly warmed up to room temperature and stirred for 30 minutes. The reaction solution was cooled down to 0°C, slowly dropwise added with a solution of 4,6-dichloro-N-(methyl-d3)pyridazine-3-formamide (3.9 g) in tetrahydrofuran (40 ml) at a controlled temperature less than 5°C, and stirred overnight after the addition. The reaction solution was added with aqueous solution of ammonium chloride (20 ml) and water (40 ml), stirred for 30 minutes, and then filtered. The filter cake was washed twice with water, and 3.95 g of an off-white solid was obtained after drying. ¹H NMR (400 MHz, CDCl₃): δ 12.53 (s, 1H), 9.39 (s, 1H), 8.38 (s, 1H), 8.34 (s, 1H), 8.22 (d, *J* = 5.2 Hz, 1H), 7.65 (d, *J* = 5.2 Hz, 1H), 5.62 (s, 2H), 4.04 (s, 3H), 3.74 (t, *J* = 8.2 Hz, 2H), 0.98 (t, *J* = 8.2 Hz, 2H), 0.02 (s, 9H). MS: m/z 494.2 [M+H]⁺.

### Step 2

The product from the previous step (2.4 g), DCPF (1.1 g), palladium acetate (111 mg), cesium carbonate (8 g), and DME (90 ml) were added into a reaction flask in sequence, heated to 90°C and reacted for 1 hour after nitrogen gas replacement. The reaction solution was cooled down to room temperature, filtered, concentrated under reduced pressure, and purified by silica gel column chromatography to obtain 2.76 g of a light yellow solid compound. MS: m/z 543.3 [M+H]⁺.

### Step 3

The product from the previous step (2.7 g) and trifluoroacetic acid (60 ml) were added into a reaction flask in sequence, stirred overnight at 70°C, concentrated under reduced pressure to dryness, added with toluene (100 ml), and then concentrated under reduced pressure to dryness again. The concentrate was added with tetrahydrofuran (100 ml), slowly added with sodium bicarbonate at room temperature to adjust the pH to 7-8, and suction filtered to remove the solid. The filtrate was directly used for the next step after concentration.

### Step 4

*N,N-*dimethylformamide (50 ml) and bromopropyne (3.6 g) were added into the product from the previous step, stirred at room temperature for 30 minutes, added with potassium carbonate (2.8 g) in batches. After the addition, the mixture was reacted for 2 h at room temperature. The reaction solution was added with 100 ml of water and extracted three times with methyl tert-butyl ether. The organic phases were combined, concentrated to dryness, and purified by silica gel column chromatography to obtain 450 mg of a light yellow solid, Compound 7. ¹H NMR (400 MHz, DMSO-*d₆*): δ 12.46 (s, 1H), 11.36 (s, 1H), 9.89 (s, 1H), 9.25 (s, 1H), 8.79 (s, 1H), 8.16 (d, *J=* 5.2 Hz, 1H), 7.50 (d, *J=* 5.2 Hz, 1H), 5.29 (d, *J=* 2.5 Hz, 2H), 3.91 (s, 3H), 3.63 (t, *J=* 2.5 Hz, 1H), 2.19 - 2.08 (m, 1H), 0.98 - 0.81 (m, 4H). MS: m/z 451.2 [M+H]⁺.

### Example 2:

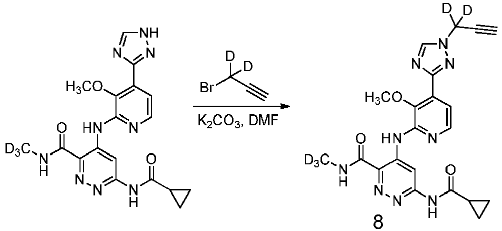

Compound 8 could be prepared by referring to the preparation scheme of Example 1. MS: m/z 453.2, [M+H]⁺. The specific preparation method was as follows:
The triazole compound (310 mg), *N, N*-dimethylformamide (45 ml) and 3-bromoprop-1-yne-3,3-*d₂* (1.2 g) were added into a 50 ml single-necked flask, and then added with potassium carbonate (310 mg) in batches under stirring. After the addition, the mixture was stirred and reacted for 1 h at room temperature. The reaction was quenched by pouring the reaction solution into 400 ml of water, and the obtained solution was extracted with 200 ml of ethyl acetate. The organic phase was separated, washed with 200 ml of water three times, dried over anhydrous sodium sulfate, filtered, concentrated and purified by silica gel column chromatography to give 120 mg of a light-yellow solid. ¹H NMR (400 MHz, DMSO-*d₆*): δ 12.47 (s, 1H), 11.37 (s, 1H), 9.90 (s, 1H), 9.26 (s, 1H), 8.80 (s, 1H), 8.16 (d, *J=* 5.2 Hz, 1H), 7.51 (d, *J* = 5.2 Hz, 1H), 3.91 (s, 3H), 3.63 (s, 1H), 2.18-2.09 (m, 1H), 0.94-0.84 (m, 4H).

### Example 3:

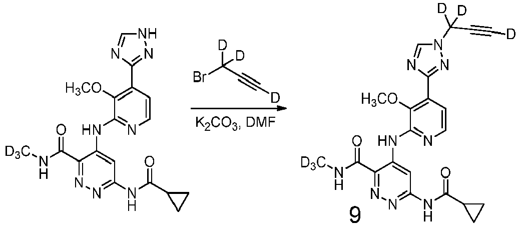

Compound 9 could be prepared by referring to the preparation scheme of Example 1. MS: m/z 454.4, [M+H]⁺. The specific preparation method was as follows:
The triazole compound (200 mg), *N, N*-dimethylformamide (15 ml) and 3-bromoprop-1-yne-1,3,3-*d₃* (1.0 g) were added into a 25 ml single-necked flask, and then added with potassium carbonate (210 mg) in batches under stirring. After the addition, the mixture was stirred and reacted for 1 h at room temperature. The reaction was quenched by pouring the reaction solution into 200 ml of water, and the obtained solution was extracted with 100 ml of ethyl acetate. The organic phase was separated, washed with 100 ml of water three times, dried over anhydrous sodium sulfate, filtered, concentrated and purified by silica gel column chromatography to give 80 mg of a light yellow solid. ¹H NMR (400 MHz, CDCl₃): δ 12.47 (s, 1H), 11.37 (s, 1H), 9.90 (s, 1H), 9.26 (s, 1H), 8.80 (s, 1H), 8.16 (d, *J=* 5.2 Hz, 1H), 7.51 (d, *J=* 5.2 Hz, 1H), 3.90 (s, 3H), 2.15-2.12 (m, 1H), 0.95-0.82 (m, 4H). HR-MS: m/z 454.2226 [M+H]⁺.

### Example 4:

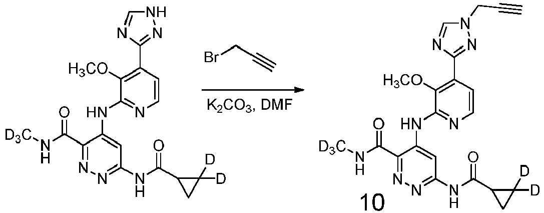

Compound 10 could be prepared by referring to the preparation scheme of Example 1. MS: m/z 453.4 [M+H]⁺.

### Example 5:

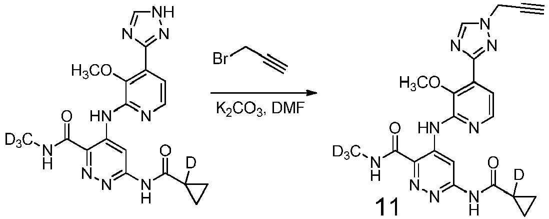

Compound 11 could be prepared by referring to the preparation scheme of Example 1. MS: m/z 452.3, [M+H]⁺. The specific preparation method was as follows:

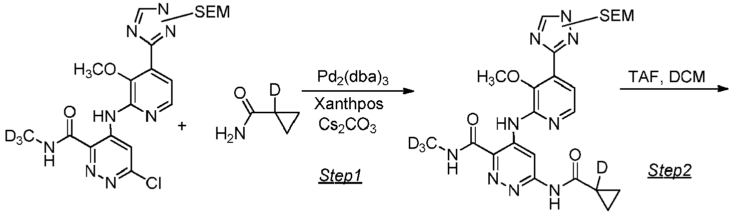

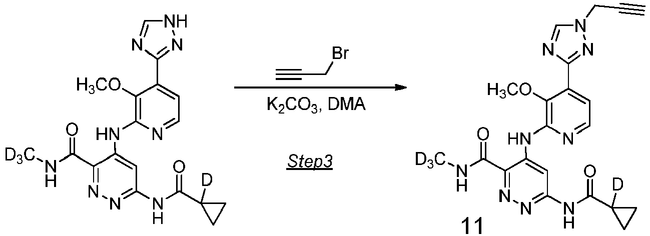

### Step 1

The triazole compound (1.20 g), cyclopropane-1-d 1-formamide (0.42 g), Xanthpos (0.28 g), Cs₂CO₃ (1.58 g), Pd₂(dba)₃ (0.22 g), and 1,4-dioxane (30 ml) were added into a reaction flask in sequence under nitrogen gas protection, heated to 100°C and stirred for reaction. After the reaction, the reaction solution was cooled down, added with water, and extracted with ethyl acetate. The organic layers were combined, and concentrated under reduced pressure to give an oil, which was then purified by silica gel column chromatography to obtain 0.74 g of a yellow solid. MS: m/z 544.3 [M+H]⁺.

### Step 2

The product from the previous step (0.72 g), dichloromethane (2.2 ml) and tetraethylammonium fluoride (2.16 g) were added into a reaction flask in sequence, and dropwise added with trifluoroacetic acid (5.04 ml) under stirring. After the addition, the resulting mixture was stirred and reacted at room temperature. After the reaction, the reaction solution was concentrated, added with water, and extracted with methyl tert-butyl ether to remove impurities. The aqueous phase was collected and the pH thereof was adjusted by using saturated sodium bicarbonate. The solid was precipitated, filtered and dried to obtain 0.44 g of a light yellow solid. MS: m/z 414.2 [M+H]⁺.

### Step 3

The product from the previous step (0.43 g), *N, N*-dimethylacetamide (20 ml) and bromopropyne (0.99 g) were added into a reaction flask in sequence, stirred until dissolution, and then added with potassium carbonate (1.01 g) in batches. The reaction was maintained at room temperature. After the reaction, the reaction solution was added with water, and extracted with ethyl acetate. The organic layers were combined, washed with water, dried, and concentrated under reduced pressure to give a yellow oil, which was then purified by silica gel column chromatography to obtain 81 mg of a white solid. ¹H NMR (400 MHz, DMSO-*d₆*): δ 12.47 (s, 1H), 11.37 (s, 1H), 9.90 (s, 1H), 9.26 (s, 1H), 8.80 (s, 1H), 8.16 (d, *J=* 5.2 Hz, 1H), 7.51 (d, *J=* 5.2 Hz, 1H), 5.29 (d, *J=* 2.6 Hz, 2H), 3.91 (s, 3H), 3.64 (t, *J=* 2.5 Hz, 1H), 0.94-0.83 (m, 4H).

Under appropriate reaction conditions, in the preparation process for the structures of Example 1, Example 5 and the like, the chemoselectivity for propargylation reaction of the triazole in the last step is superior to that of methyl and other substituents; and also superior to the chemoselectivity for the corresponding binding reaction of the nitrogen atom in the triazole when "A ring" in the general formula (I) are similar structures to other ring systems. The obtained compound products are easy to be crystallized. The above features are conducive to post-process and purification.

### Example 6:

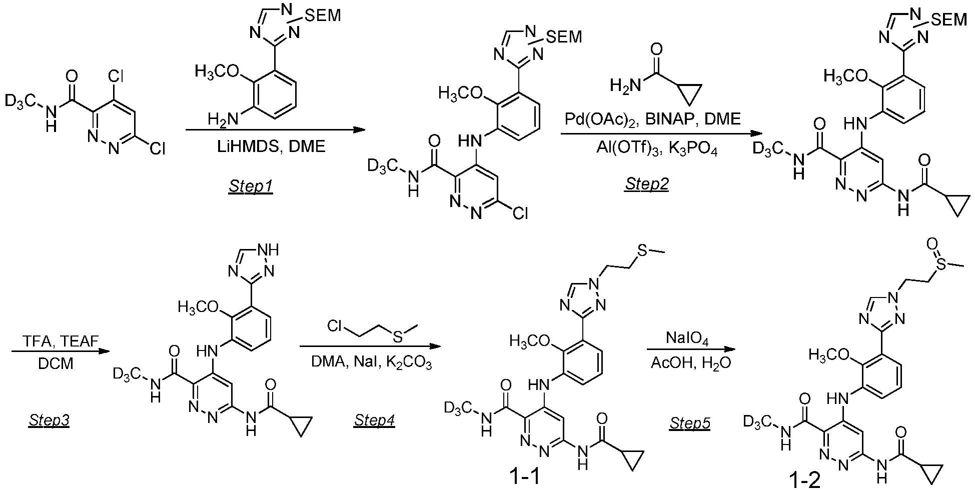

### Step 1

4,6-dichloro-*N-*(methyl-*d₃*)pyridazine-3-formamide (9.66 g), the triazole compound (10.0 g), and ethylene glycol dimethyl ether (200 ml) were added into a 500 ml single-necked flask, stirred until dissolution, cooled down to 10°C to 20°C, and dropwise added with LiHMDS (1M in THF, 110 ml). After the addition, the mixture was stirred for 2 h at room temperature until the reaction was monitored by TLC to be completed. The reaction solution was poured into 300 ml of saturated aqueous solution of ammonium chloride, and the resulting solution was extracted with ethyl acetate and separated. The separated solution was dried over anhydrous sodium sulfate, filtered and concentrated under reduced pressure. The residue was added with 500 ml of methyl tert-butyl ether to precipitate a solid, and then filtered. The filtrate was concentrated to dryness, and the obtained concentrate was then added with 500 ml of a solvent consisting of n-hexane and ethyl acetate at a ratio of 4:1. The resulting solution was stood overnight for crystallization and 5.3 g of a light-yellow solid was obtained.

### Step 2

The product from the previous step (4.0 g), cyclopropanecarboxamide (1.4 g), potassium phosphate (5.16 g), BINAP (2.0 g), palladium acetate (182 mg), aluminum trifluoromethanesulfonate (192 mg) and ethylene glycol dimethyl ether (150 ml) were added into a 250 ml single-necked flask. After nitrogen gas replacement, the mixture was warmed up to 90°C for overnight reaction. The reaction solution was cooled down to room temperature, and poured into 500 ml of water. The resulting solution was extracted with ethyl acetate and separated. The separated solution was dried and concentrated to give 6.5 g of a crude product, which was directly used for the next step.

### Step 3

The crude product from the previous step (6.5 g), dichloromethane (70 ml), and tetraethylammonium fluoride (4.2 g) were added into a 250 ml single-necked flask, stirred for 10 minutes, then added with trifluoroacetic acid (60 ml) and stirred overnight at room temperature. The reaction solution was concentrated under reduced pressure, and the residue was poured into 100 ml of saturated aqueous solution of sodium bicarbonate. The resulting solution was extracted with ethyl acetate, dried over anhydrous sodium sulfate, filtered, and concentrated under reduced pressure to dryness. The concentrate was triturated with 50 ml of ethyl acetate to yield 3.5 g of a yellow solid. ¹H NMR (400 MHz, DMSO-*d₆*): δ = 11.36 (br s, 1H), 11.00 (s, 1H), 9.17 (s, 1H), 8.32 (s, 1H), 8.16 (s, 1H), 7.76 (dd, *J* = 7.9, 1.5 Hz, 1H), 7.55 (dd, *J* = 8.0, 1.5 Hz, 1H), 7.31 (t, *J* = 7.9 Hz, 1H), 3.69 (s, 3H), 2.14-2.02 (m, 1H), 0.88-0.75 (m, 4H). ¹³C NMR (100 MHz, DMSO-*d₆*) δ = 174.2, 167.0, 156.3, 154.5, 150.9, 148.6, 145.2, 135.5, 132.6, 126.4, 125.1, 124.7, 124.0, 97.2, 61.6, 14.9, 8.6.

### Step 4

The product from the previous step (1.3 g), 2-chloroethylmethylsulfide (1.4 g), *N,N*-dimethylacetamide (30 ml), potassium carbonate (1.1 g), and sodium iodide (100 mg) were added into a 50 ml single-necked flask. After the addition, the mixture was heated to 100°C and reacted for 1 h. The reaction solution was poured into 500 ml of water, and the resulting solution was extracted with ethyl acetate. The organic phases were combined, washed with water three times, dried over anhydrous sodium sulfate, filtered, concentrated under reduced pressure and purified by silica gel column chromatography to give 400 mg of a light yellow solid. ¹H NMR (400 MHz, DMSO-*d₆*): δ = 11.35 (s, 1H), 11.01 (s, 1H), 9.16 (s, 1H), 8.66 (s,1H), 8.18 (s, 1H), 7.67 (dd, *J=* 7.8, 1.5 Hz, 1H), 7.53 (dd, *J=* 7.9, 1.4 Hz, 1H), 7.30 (t, *J=* 7.9 Hz, 1H), 4.46 (t, *J=* 6.5 Hz, 2H), 3.72 (s, 3H), 2.99 (t, *J=* 6.6 Hz, 2H), 2.13-2.06 (m, 1H), 2.05 (s, 3H), 0.87-0.79 (m, 4H). ¹³C NMR (100 MHz, DMSO-*d₆*): δ = 174.2, 167.0, 159.4, 156.3, 151.0, 145.6, 145.2, 135.5, 133.0, 126.6, 126.5, 124.8, 123.2, 97.2, 61.6, 48.6, 33.4, 14.8, 8.6. MS: m/z 486.2 [M+H]⁺.

### Step 5

The product from the previous step (360 mg), acetic acid (10 ml), NaIO₄ (500 mg), and 2 drops of water were added into a 50 ml single-necked flask. After the addition, the mixture was heated to 50°C and reacted for 2 h. The reaction solution was poured into 100 ml of saturated aqueous solution of sodium bicarbonate. The resulting solution was extracted with ethyl acetate, dried over anhydrous sodium sulfate, filtered, concentrated under reduced pressure and purified by silica gel column chromatography to yield 250 mg of a light-yellow solid. ¹H NMR (400 MHz, DMSO-*d₆*): δ = 11.35 (s, 1H), 11.01 (s, 1H), 9.16 (s, 1H), 8.70 (s,1H), 8.17 (s, 1H), 7.67 (dd, *J* = 7.9, 1.6 Hz, 1H), 7.53 (dd, *J* = 8.0, 1.5 Hz, 1H), 7.29 (t, *J* = 7.9 Hz, 1H), 4.68 (t, *J* = 6.6 Hz, 2H), 3.73 (s, 3H), 3.38 (t, *J* = 6.6 Hz, 2H), 2.62 (s, 3H), 2.13-2.03 (m, 1H), 0.89-0.75 (m, 4H). ¹³C NMR (100 MHz, DMSO-*d₆*): δ = 174.2, 167.0, 159.6, 156.3, 151.0, 145.6, 145.2, 135.5, 133.0, 126.51, 126.48, 124.8, 123.3, 97.2, 61.6, 52.6, 43.2, 38.6, 14.9, 8.6. MS: m/z 502.2 [M+H]⁺.

### Example 7:

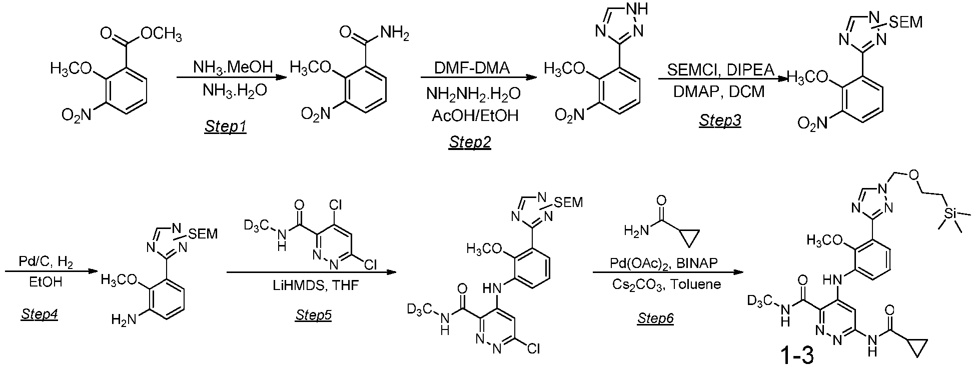

### Step 1

Methyl 2-methoxy-3-nitrobenzoate (55 g), ammonia in methanol solution (7 M, 1200 ml), and ammonia water (500 ml) were added into a 2000 ml single-necked flask. After the addition, the mixture was stirred for 17 h at room temperature. After the reaction, the reaction solution was concentrated to dryness, triturated with 1000 ml of water for 10 minutes and filtered. The filter cake was washed with water twice, collected, and dried. 50 g of a yellow solid was obtained. ¹H NMR (400 MHz, CDCl₃): δ 8.27 (dd, *J* = 7.9, 1.8 Hz, 1H), 7.95 (dd, *J* = 8.1, 1.8 Hz, 1H), 7.43 (br s, 1H), 7.36 (t, *J=* 8.0 Hz, 1H), 6.85 (br s, 1H), 4.01 (s, 3H). MS: m/z 197.1 [M+H]⁺.

### Step 2

2-methoxy-3-nitro-benzamide (50 g) and *N,N-*dimethylformamide dimethyl acetal (DMF-DMA, 300 ml) were added into a 3000 ml single-necked flask. After the addition, the mixture was stirred for 1 h at 95°C, concentrated to dryness, and subjected to azeotropy twice with 1,2-dichloroethane. The concentrate was added with anhydrous ethanol (2000 ml) and acetic acid (250 ml), and slowly dropwise added with hydrazine hydrate (120 ml) under ice bath. After the addition, the resulting mixture was stirred for another 6 h at room temperature. After the reaction, the reaction solution was concentrated to dryness, added with 1000 ml of water, stirred for 10 minutes and filtered. The filter cake was washed with water and collected. 55 g of a yellow solid was obtained after drying. ¹H NMR (400 MHz, DMSO-*d₆*): δ 8.55 (s, 1H), 8.22 (dd, *J* = 7.9, 1.6 Hz, 1H), 7.99 (dd, *J=* 8.0, 1.4 Hz, 1H), 7.46 (t, *J=* 8.0 Hz, 1H), 3.81 (s, 3H). MS: m/z 221.1 [M+H]⁺.

### Step 3

The product from the previous step (10 g), *N,N-*diisopropylethylamine (8.2 g), 4-dimethylaminopyridine (55.5 mg), and dichloromethane (150 ml) were added into a 500 ml single-necked flask, and then dropwise added with 2-(trimethylsilyl)ethoxymethyl chloride (9.1 g) at room temperature. After the addition, the mixture was stirred at room temperature for 3 h. When the reaction was monitored by TLC to be approximately half completed, the reaction solution was filtered and concentrated under reduced pressure, and the residue was dissolved using 200 ml of ethyl acetate and washed with water three times (200 ml/time). The organic phase was dried over anhydrous sodium sulfate, filtered, concentrated under reduced pressure, and purified by silica gel column chromatography to give 5.3 g of a yellow oil. The product was a mixture of two isomers with a ratio of approximately 62:38. The hydrogen spectrum of the isomer with higher content was as follows: ¹H NMR (400 MHz, CDCl₃): δ 8.36 (s, 1H), 8.26 (dd, *J* = 7.8, 1.7 Hz, 1H), 7.83 (dd, *J* = 8.1, 1.7 Hz, 1H), 7.32 (t, *J* = 8.0 Hz, 1H), 5.60 (s, 2H), 3.96 (s, 3H), 3.73 (t, *J=* 8.2 Hz, 2H), 0.97 (t, *J=* 8.3 Hz, 2H), 0.01 (s, 9H). MS: m/z 351.2 [M+H]⁺.

### Step 4

The product from the previous step (5.3 g), ethanol (150 ml) and 5% palladium on carbon (530 mg) were added into a 500 ml single-necked flask. After the addition, the atmosphere in the flask was replaced with hydrogen gas sufficiently, and the mixture was reacted for 3 h at room temperature. After the reaction, the reaction solution was filtered through a celite pad and the filtrate was concentrated to give 4.8 g of a yellow oil. MS: m/z 321.2 [M+H]⁺.

### Step 5

The product from the previous step (4.8 g),
4,6-dichloro-*N-*(methyl-*d₃*)pyridazine-3-formamide (2.97 g), and tetrahydrofuran (20 ml) were added into a 250 ml single-necked flask. After nitrogen replacement, the mixture was slowly dropwise added with lithium bis(trimethylsilyl)amide (35.7 ml) under ice bath. After the addition, the reaction was continued for 0.5 h until it was completed. The reaction solution was poured into 50 ml of aqueous solution of ammonium chloride, then added with 200 ml of water and 200 ml of ethyl acetate, stirred, stood, and separated. The separated solution was dried over anhydrous sodium sulfate, filtered, concentrated under reduced pressure, and purified by silica gel column chromatography to yield 5 g of a yellow oil. MS: m/z 493.2 [M+H]⁺.

### Step 6

The product from the previous step (5 g), cyclopropanecarboxamide (1.2 g), cesium carbonate (19 g), BINAP (1.6 g), toluene (150 ml) and palladium acetate (262 mg) were added into a 500 ml single-necked flask. After the addition, the atmosphere in the flask was replaced with nitrogen gas sufficiently, and the mixture was reacted for 1.5 h at 90°C until the reaction was analyzed to be completed. The reaction solution was cooled down to room temperature, diluted with 200 ml of water, and extracted with ethyl acetate three times. The organic phases were combined, dried over anhydrous sodium sulfate, filtered, concentrated under reduced pressure, and purified by silica gel column chromatography to yield 3 g of a yellow oil. MS: m/z 542.3 [M+H]⁺.

### Example 8:

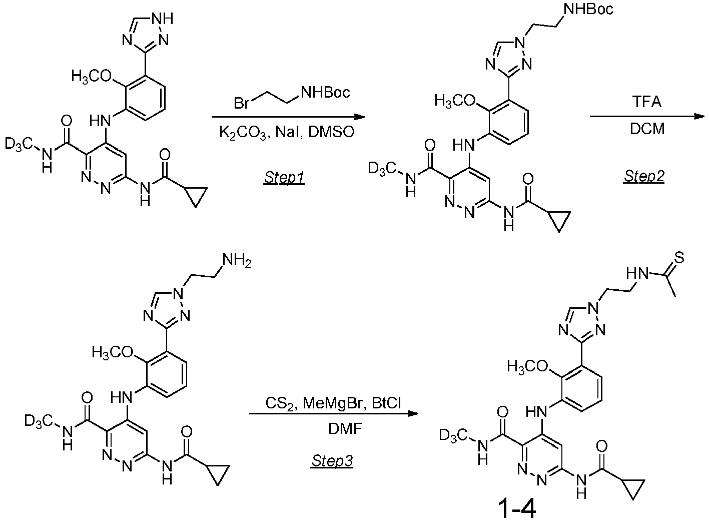

### Step 1

6-(cyclopropylamide)-4-((2-methoxy-3-(1*H*-1,2,4-triazol-3-yl)phenyl)amino)-*N-*(methyl-*d₃*) pyridazine-3-formamide (3 g), 2-(Boc-amino)-bromoethane (4.8 g), potassium carbonate (2.18 g), sodium iodide (200 mg), and DMSO (70 ml) were added into a 100 ml single-necked flask. After the addition, the mixture was heated to 100°C and stirred for 2 h. After the reaction, the reaction solution was cooled down to room temperature, added with water for quenching, and extracted with ethyl acetate. The organic layers were combined, washed with water three times, dried over anhydrous sodium sulfate, and filtered. The filtrate was concentrated and purified by silica gel column chromatography to obtain 800 mg of a light yellow solid, with a yield of 19%. MS: m/z 555.3 [M+H]⁺.

### Step 2

The product from the previous step (800 mg) and dichloromethane (20 ml) were added into a 100 ml single-necked flask, then slowly dropwise added with TFA (10 ml), and stirred at room temperature for 2 h. After the reaction, the reaction solution was concentrated under reduced pressure, and the concentrate was added with saturated aqueous solution of sodium bicarbonate and extracted with ethyl acetate. The organic layers were combined, washed with saturated brine once, dried over anhydrous sodium sulfate, and filtered. The filtrate was concentrated and purified by silica gel column chromatography (ethyl acetate) to obtain 600 mg of an off-white solid.

### Step 3

Carbon disulfide (0.5 g) and tetrahydrofuran (50 ml) were added into a 100 ml single-necked flask. After nitrogen replacement, the mixture was slowly dropwise added with methyl magnesium bromide (1M) at room temperature, heated to 65°C after the addition, and stirred for 1 h. After the reaction, the reaction solution was cooled down to room temperature, added with 1-chlorobenzotriazole (1 g), and stirred for another 20 minutes at room temperature for use.

The product from the previous step (350 mg) and DMF (35 ml) were added into a 50 ml single-necked flask, then slowly dropwise added with the above stand-by reagent (9 ml) at room temperature, and stirred for 30 minutes. After the reaction, the reaction solution was slowly poured into water for quenching, and extracted with ethyl acetate. The organic layers were combined, washed once with saturated brine, dried over anhydrous sodium sulfate, and filtered. The filtrate was concentrated and purified by silica gel column chromatography to obtain 180 mg of a reddish brown solid. ¹H NMR (400 MHz, CDCl₃): δ 10.99 (s, 1H), 9.86 (s, 1H), 8.70 (s, 1H), 8.17-8.10 (m, 3H), 7.83 (dd, *J* = 7.9, 1.5 Hz, 1H), 7.52 (dd, *J=* 7.9, 1.4 Hz, 1H), 7.29 (t, *J* = 7.9 Hz, 1H), 4.57-4.54 (m, 2H), 4.22-4.18 (m, 2H), 3.83 (s, 3H), 2.55 (s, 3H), 1.87-1.81 (m, 1H), 1.09-1.05 (m, 2H), 0.95-0.88 (m, 2H). ¹³C NMR (100 MHz, CDCl₃): δ 202.4, 173.6, 166.8, 160.5, 155.7, 151.4, 146.0, 144.2, 134.8, 132.2, 127.2, 125.7, 124.9, 124.3, 98.1, 61.5, 47.0, 45.1, 34.0, 16.0, 8.9. MS: m/z 513.2[M+H]⁺.

### Example 9:

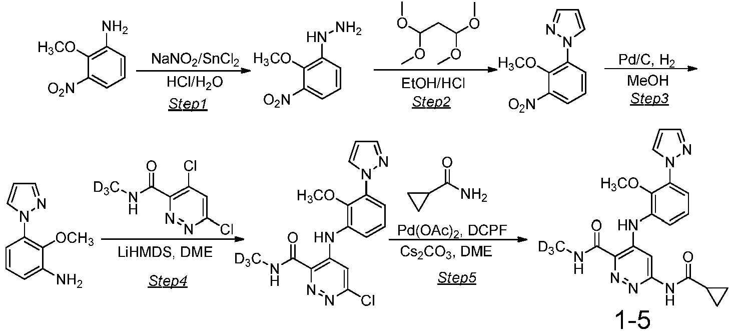

### Step 1

Concentrated hydrochloric acid (40 ml) and water (40 ml) were added into a 500 ml three-necked flask, then slowly dropwise added with an aqueous solution (20 ml) of sodium nitrite (2.1 g, 30.4 mmol) under ice bath, and stirred at 0-5°C for 1 h. Then the mixture was slowly dropwise added with a solution of stannous chloride (17.1 g, 90.2 mmol) in concentrated hydrochloric acid (20 ml) and stirred at 0-5°C for 2 h. After the reaction, the reaction solution was filtered through a celite pad, and the filtrate was adjusted to a pH of around 8 by using saturated aqueous solution of sodium hydroxide, and extracted with ethyl acetate. The organic layers were combined, washed once with saturated brine, dried over anhydrous sodium sulfate, and filtered. The filtrate was concentrated to dryness, added with methyl tert-butyl ether (30 ml), stirred at room temperature for 20 minutes, and filtered. The filter cake was dried to obtain 2.4 g of a yellow solid. MS: m/z 184.1 [M+H]⁺.

### Step 2

The product from the previous step (2.4 g), 1,1,3,3-tetramethoxypropane (2.6 g), and anhydrous ethanol (60 ml) were added into a 100 ml single-necked flask. The resulting mixture was heated to 80°C, stirred for 2 h, then slowly dropwise added with concentrated hydrochloric acid (1.5 ml), and stirred at 80°C for 2 h. After the reaction, the reaction solution was cooled down to room temperature, concentrated to dryness, slowly added with saturated aqueous solution of NaHCO₃ to adjust the pH of aqueous phase to 7-8, and extracted with ethyl acetate. The organic layers were combined, washed once with saturated brine, dried over anhydrous sodium sulfate, and filtered. The filtrate was concentrated and purified by silica gel column chromatography to obtain 2.8 g of a pink solid. MS: m/z 220.1 [M+H]⁺.

### Step 3

The product from the previous step (2.8 g, 12.8 mmol), 5% Pd/C (1 g), and methanol (60 ml) were added into a 100 ml single-necked flask. After hydrogen replacement, the mixture was stirred for 5 h at room temperature. After the reaction, the reaction solution was filtered through a celite pad and the filtrate was concentrated to give 1.8 g of a pink solid. MS: m/z 190.1 [M+H]⁺.

### Step 4

The product from the previous step (1.8 g, 9.6 mmol), 4,6-dichloro-*N-*(methyl-*d₃*)pyridazine-3-formamide (3.0 g, 14.4 mmol), and ethylene glycol dimethyl ether (70 ml) were added into a 250 ml three-necked flask. After nitrogen gas replacement, the mixture was cooled down to 10-15°C, slowly dropwise added with LiHMDS (1M in THF, 38.4 ml), and reacted for 30 minutes at room temperature. After the reaction, the reaction solution was added with saturated aqueous solution of ammonium chloride for quenching and extracted with ethyl acetate. The extracted solution was washed once with saturated brine, dried over anhydrous sodium sulfate, and filtered. The filtrate was concentrated to dryness, added with methyl tert-butyl ether (60 ml), stirred at room temperature for 20 minutes, and filtered. The filter cake was dried to obtain 2.8 g of a light-yellow solid. MS: m/z 362.1 [M+H]⁺.

### Step 5

The product from the previous step (1.5 g), cyclopropanecarboxamide (530 mg), cesium carbonate (6.8 g), ethylene glycol dimethyl ether (60 ml), 1,1'-di(dicyclohexylphosphino)-ferrocene (961 mg), and palladium acetate (95 mg) were added into a 100 ml single-necked flask, heated to 90°C and stirred for 2 h. After the reaction, the reaction solution was cooled down to room temperature, added with water, and extracted with ethyl acetate. The extracted solution was washed once with saturated brine, dried over anhydrous sodium sulfate, and filtered. The filtrate was concentrated to dryness, added with methyl tert-butyl ether (60 ml), stirred at room temperature for 20 minutes, and filtered. The filter cake was dissolved in dichloromethane (20 ml), then slowly added with methyl tert-butyl ether (80 ml), stirred at room temperature for 20 minutes, and filtered. The obtained filter cake was dried to give 950 mg of an off-white solid. ¹H NMR (400 MHz, DMSO-*d₆*): δ 11.38 (s, 1H), 11.07 (s, 1H), 9.18 (s, 1H), 8.22 (d, *J* = 2.4 Hz, 1H), 8.21 (s, 1H), 7.78 (d, *J* = 1.7 Hz, 1H), 7.47 (td, *J* = 8.5, 1.6 Hz, 2H), 7.32 (t, *J=* 8.1 Hz, 1H), 6.57 (t, *J* = 2.1 Hz, 1H), 3.46 (s, 3H), 2.14-2.04 (m, 1H), 0.88-0.79 (m, 4H). ¹³C NMR (100 MHz, DMSO-*d₆*): δ 174.2, 167.0, 156.4, 145.2, 144.9, 141.1, 135.5, 134.8, 133.1, 131.9, 125.2, 121.6, 121.3, 107.8, 97.5, 61.2, 14.9, 8.6. MS: m/z 411.2 [M+H]⁺.

### Example 10:

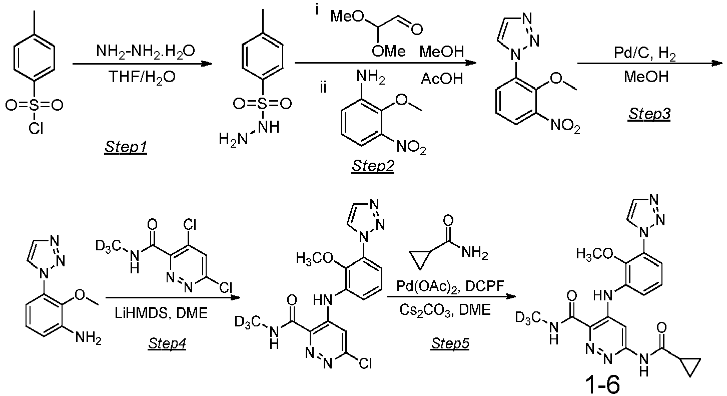

### Step 1

80% hydrazine hydrate (32.8 g) and water (60 ml) were added into a 250 ml three-necked flask, then slowly dropwise added with a tetrahydrofuran solution (30 ml) of *p*-methylbenzenesulfonyl chloride (10.0 g) under ice bath, and stirred at room temperature for 30 minutes. After the reaction, the reaction solution was concentrated to remove tetrahydrofuran, cooled down to room temperature, and filtered. The filter cake was dried to give 8.3 g of a white solid. MS: m/z 187.1 [M+H]⁺.

### Step 2

The product from the previous step (4.8 g), 2,2-dimethoxyacetaldehyde (3.6 g), and methanol (60 ml) were added into a 100 ml single-necked flask. After nitrogen gas replacement, the mixture was stirred at room temperature for 3 h, then added with acetic acid (1.3 g) and 2-methoxy-3-nitroaniline (3 g), heated to 75°C, and stirred for 16 h. After the reaction, the reaction solution was concentrated to dryness, slowly added with saturated aqueous solution of NaHCO₃ to adjust the pH of the aqueous phase to 7-8, and extracted with ethyl acetate. The organic layers were combined, washed once with saturated brine, dried over anhydrous sodium sulfate, and filtered. The filtrate was concentrated and purified by silica gel column chromatography to obtain 3.8 g of an off-white solid. ¹H NMR (400 MHz, DMSO-*d₆*): δ 8.63 (d, *J=* 1.2 Hz, 1H), 8.16 (dd, *J=* 8.2, 1.6 Hz, 1H), 8.05 (d, *J* = 1.1 Hz, 1H), 8.03 (dd, *J=* 8.1, 1.6 Hz, 1H), 7.57 (t, *J=* 8.2 Hz, 1H), 3.53 (s, 3H). MS: m/z 221.1 [M+H]⁺.

### Step 3

The product from the previous step (3.7 g), 5% Pd/C (2 g) and methanol (70 ml) were added into a 100 ml single-necked flask. After hydrogen replacement, the mixture was stirred for 5 h at room temperature. After the reaction, the reaction solution was filtered through a celite pad and the filtrate was concentrated to obtain 3.0 g of an off-white solid. MS: m/z 191.1 [M+H]⁺.

### Step 4

The product from the previous step (3.0 g), 4,6-dichloro-*N-*(methyl-*d₃*)pyridazine-3-formamide (5.0 g) and ethylene glycol dimethyl ether (80 ml) were added into a 250 ml three-necked flask. After nitrogen gas replacement, the mixture was cooled down to 10-15°C, slowly dropwise added with LiHMDS (1M in THF), and reacted for 30 minutes at room temperature. After the reaction, the reaction solution was added with saturated aqueous solution of ammonium chloride for quenching and extracted with ethyl acetate. The extracted solution was washed once with saturated brine, dried over anhydrous sodium sulfate, and filtered. The filtrate was concentrated to dryness, added with methyl tert-butyl ether (100 ml), stirred at room temperature for 20 minutes, and filtered. The filter cake was dried to obtain 4.6 g of a yellow solid. MS: m/z 363.1 [M+H]⁺.

### Step 5

The product from the previous step (1.5 g), cyclopropanecarboxamide (529 mg), cesium carbonate (6.8 g), ethylene glycol dimethyl ether (60 ml), 1,1'-di(dicyclohexylphosphino)-ferrocene (961 mg), and palladium acetate (95 mg) were added into a 100 ml single-necked flask, heated to 90°C and stirred for 2 h. After the reaction, the reaction solution was cooled down to room temperature, added with water, and extracted with ethyl acetate. The extracted solution was washed once with saturated brine, dried over anhydrous sodium sulfate, and filtered. The filtrate was concentrated and purified by silica gel column chromatography to obtain 230 mg of an off-white solid. ¹H NMR (400 MHz, DMSO-*d₆*): δ 11.41 (s, 1H), 11.09 (s, 1H), 9.20 (s, 1H), 8.56 (s, 1H), 8.22 (s, 1H), 8.01 (s, 1H), 7.66 (d, *J* = 8.0 Hz, 1H), 7.49 (d, *J=* 8.1 Hz, 1H), 7.41 (t, *J* = 8.0 Hz, 1H), 3.47 (s, 3H), 2.14-2.05 (m, 1H), 0.90-0.78 (m, 4H). ¹³C NMR (100 MHz, DMSO-*d₆*): δ 174.3, 167.0, 156.4, 146.0, 144.8, 135.5, 134.3, 133.3, 131.8, 127.1, 125.5, 123.6, 122.4, 97.5, 61.7, 14.9, 8.7. MS: m/z 412.2 [M+H]⁺.

### Example 11:

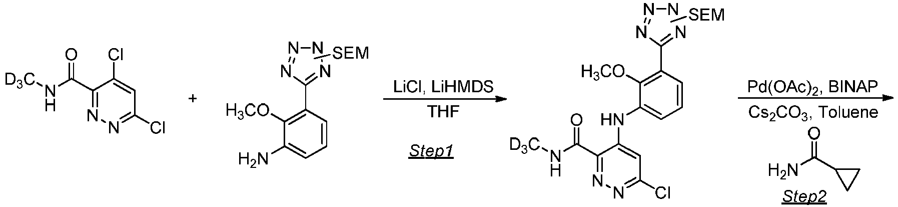

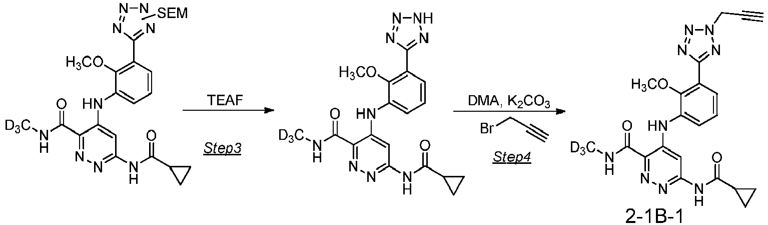

### Step 1

The tetrazole compound (18 g), 4,6-dichloro-*N*-(methyl-*d₃*)pyridazine-3-fortnamide (15 g), lithium chloride (5.8 g) and dry tetrahydrofuran (350 ml) were added into a 500 ml single-necked flask, inside which nitrogen gas replacement was performed after the addition. The resulting mixture was cooled down to 0°C by ice bath, dropwise added with lithium bis(trimethylsilyl)amide (150 ml), naturally warmed up to room temperature after the addition, and reacted for 2 h. The reaction solution was poured into 500 ml of saturated aqueous solution of ammonium chloride, and extracted with ethyl acetate (500 ml). The extracted solution was dried over anhydrous sodium sulfate, filtered, and concentrated under reduced pressure. The concentrate was added with 200 ml of dichloromethane for dissolution, triethylamine (3 g) and triphenylchloromethane (8 g), and stirred at room temperature for 30 minutes to remove the unreacted tetrazole raw material. After the reaction, the reaction solution was washed with 200 ml of water and then separated. The organic phase was dried over anhydrous sodium sulfate, filtered, concentrated under reduced pressure, and purified by silica gel column chromatography to give 19.0 g of a light yellow oil.

### Step 2

The product from the previous step (19 g), cyclopropanecarboxamide (6.6 g), BINAP (9.6 g), cesium carbonate (62.8 g) and toluene (450 ml) were added into a 500 ml single-necked flask. After the addition, the atmosphere in the flask was replaced with nitrogen gas sufficiently, and the mixture was heated up to 60°C, then added with palladium acetate (865 mg), warmed up again to 90°C and reacted for 5 h. The residual raw materials were monitored by TLC. The reaction solution was poured into 500 ml of water, and extracted with ethyl acetate (300 ml). The organic phase was purified by silica gel column chromatography after concentration to obtain 10 g of a yellow solid.

### Step 3

Tetraethylammonium fluoride dihydrate (100 g) was added into a 250 ml single-necked flask, heated to 85°C for melting, added with the product from the previous step (10 g), and reacted for 3 h at the same temperature. The residual raw materials were monitored by TLC. The reaction solution was poured into 500 ml of water, stirred for 20 minutes to precipitate a large amount of solid, and filtered. The filter cake was washed with 200 ml of water, then transferred to a 250 ml single-necked flask, added with 100 ml of ethyl acetate, stirred at room temperature for 30 minutes, and filtered. The resulting filter cake was dried to obtain 5 g of a light yellow solid.

### Step 4

The product from the previous step (2.5 g), *N,N-*dimethylacetamide (50 ml), and bromopropyne (5.7 g) were added into a 100 ml single-necked flask. After the addition, the mixture was stirred for 20 minutes at room temperature, then added with potassium carbonate (5.9 g) in batches. After the addition, the resulting mixture was stirred and reacted for 3 h at room temperature until the reaction was monitored by TLC to be completed. The reaction solution was poured into 500 ml of ice water and extracted twice with 200 ml of ethyl acetate. The organic phase was washed with 300 ml of water three times, dried over anhydrous sodium sulfate, filtered, and concentrated under reduced pressure to give 1.5 g of a crude product, which was then purified by silica gel column chromatography and triturated with ethyl acetate (50 ml) to obtain 650 mg of an off-white solid. ¹H NMR (400 MHz, DMSO-*d₆*): δ 11.38 (s, 1H), 11.06 (s, 1H), 9.18 (s, 1H), 8.18 (s, 1H), 7.73 (dd, *J* = 7.8, 1.5 Hz, 1H), 7.67 (dd, *J* = 8.0, 1.4 Hz, 1H), 7.39 (t, *J=* 7.9 Hz, 1H), 5.83 (d, *J=* 2.6 Hz, 2H), 3.77 (t, *J=* 5.2 Hz, 1H), 3.75 (s, 3H), 2.15-2.03 (m, 1H), 0.90-0.75 (m, 4H). ¹³C NMR (100 MHz, DMSO-*d₆*): δ 174.2, 167.0, 162.5, 156.3, 151.2, 145.0, 135.5, 133.3, 126.3, 125.5, 125.0, 122.4, 97.3, 78.8, 76.0, 61.9, 43.2, 14.9, 8.6. MS: m/z 451.2 [M+H]⁺.

### Example 12:

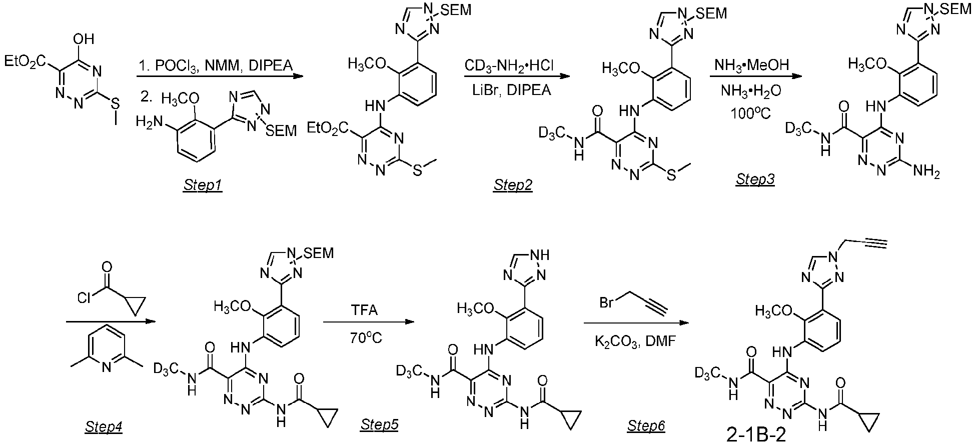

### Step 1

Ethyl 5-hydroxy-3-(methylthio)-1,2,4-triazine-6-carboxylate (4 g) and tetrahydrofuran (70 ml) were added into a reaction flask, and cooled down to 0°C. The mixture was slowly added with DIPEA (6.5 ml) and NMM (94 mg) in sequence, and then dropwise added with phosphorus oxychloride (2.6 ml) while the temperature was controlled to be less than 3°C. After the addition, the reaction was carried out for 1 h. The reaction solution was added with n-hexane and stirred for 10 minutes, then filtered through a celite pad. The filter cake was washed with n-hexane once, and the filtrate was concentrated to obtain 3.13 g of a light yellow solid.

The concentrate was added with NMP (30 ml) and the triazole-SEM compound (4.7 g), and the obtained mixture was reacted for 1 h at room temperature. The reaction solution was added with saturated ammonium chloride (10 ml) and water (20 ml), stirred for 30 minutes under ice bath, and filtered. The filter cake was flushed twice with water, and dried to give 6.9 g of a light yellow solid. MS: m/z 518.2 [M+H]⁺.

### Step 2

The product from the previous step (6.9 g), deuterated methylamine hydrochloride (1.32 g, 18.7 mmol), lithium bromide (4.6 g), acetonitrile (120 ml), and DIPEA (12 ml) were added into a reaction flask in sequence. After nitrogen gas replacement, the mixture was stirred at room temperature for 45 minutes. The reaction solution was added with saturated ammonium chloride (50 ml) and extracted with ethyl acetate (30 × 3). The extracted solution was washed with saturated aqueous sodium chloride, dried over anhydrous sodium sulfate, filtered, and concentrated to obtain 4.65 g of a light yellow solid. MS: m/z 506.2 [M+H]⁺.

### Step 3

The product from the previous step (4.5 g), ammonia in methanol solution (7M) and ammonia water (70 ml) were added into a reaction flask. After the addition, the mixture was slowly warmed up to 110°C and reacted for 6 h. The reaction solution was concentrated to a small volume and filtered. The filtrate was extracted twice with dichloromethane, and the filter cake solids were combined and dissolved in dichloromethane. The dichloromethane solutions were combined, dried over anhydrous sodium sulfate, filtered, and concentrated to obtain 4.2 g of a yellow solid. MS: m/z 475.2 [M+H]⁺.

### Step 4

The product from the previous step (4.2 g), dichloromethane (80 ml), and 2.6-dimethylpyridine (5.7 g) were added into a reaction flask in sequence. After nitrogen gas replacement, the mixture was cooled down to 0°C and slowly dropwise added with cyclopropanecarbonyl chloride (2.3 g). After the addition, the reaction was carried out for 30 minutes. The reaction solution was added with saturated ammonium chloride for quenching and extracted with MTBE three times. The organic phases were combined, washed twice with saturated brine, dried over anhydrous sodium sulfate, and filtered. The filtrate was concentrated to dryness, and purified by silica gel column chromatography to obtain 4.2 g of a light yellow solid. MS: m/z 543.3 [M+H]⁺.

### Step 5

The product from the previous step (4.2 g), dichloromethane (4 ml), and TFA (80 ml) were added into a reaction flask. The obtained mixture was heated to 40°C and reacted for 30 minutes. The reaction solution was concentrated under reduced pressure to dryness, added with THF (80 ml) and sodium bicarbonate (20 g), stirred until no bubbles were released, and then filtered. The filtrate was dried, concentrated, and directly used for the next step.

### Step 6

The product from the previous step, DMF (40 ml) and potassium carbonate (2.1 g) were added into a reaction flask. The mixture was dropwise added with bromopropyne (7.4 g) at room temperature, then reacted for 1 h at the same temperature after the addition. The reaction solution was added with water for quenching and extracted with MTBE three times. The organic phases were combined, washed once with saturated brine, dried over anhydrous sodium sulfate, and filtered. The filtrate was concentrated to dryness, purified by silica gel column chromatography, and then purified by preparative liquid chromatography to give 134 mg of a light yellow solid. ¹H NMR (400 MHz, DMSO-*d₆*): δ 12.15 (s, 1H), 11.43 (s, 1H), 9.29 - 9.18 (m, 2H), 8.70 (s, 1H), 7.60 (dd, *J* = 7.8, 1.4 Hz, 1H), 7.23 (t, *J* = 8.1 Hz, 1H), 5.24 (d, *J* = 2.4 Hz, 2H), 3.84 (s, 3H), 3.61 (t, *J=* 2.5 Hz, 1H), 2.25 - 2.14 (m, 1H), 0.98 - 0.87 (m, 4H). MS: m/z 451.2 [M+H]⁺.

### Example 13:

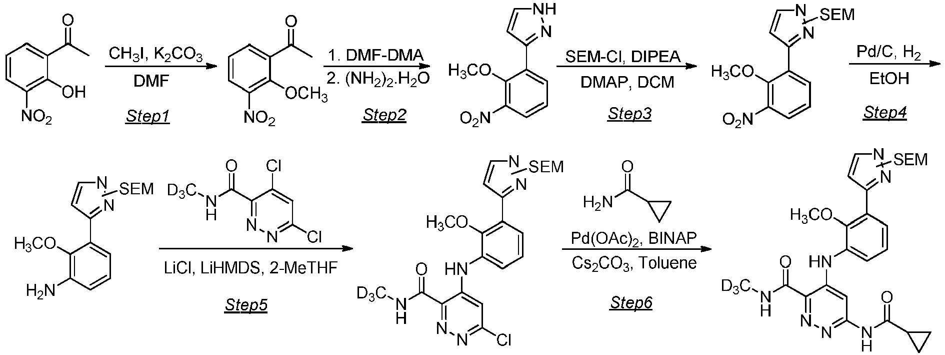

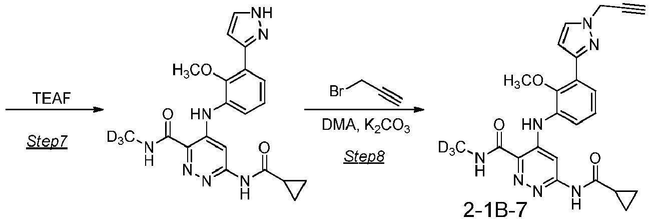

### Step 1

2-hydroxy-3-nitroacetophenone (30 g), potassium carbonate (46 g), and *N,N*-dimethylformamide (300 ml) were added into a 1000 ml three-necked flask. After the addition, the mixture was stirred at room temperature, then slowly dropwise added with iodomethane (38.4 g), reacted for 20 minutes at room temperature after the addition, and then heated to 50°C and stirred overnight. After the reaction, the system was cooled down to room temperature, added with saturated brine (500 ml), and extracted three times with methyl tert-butyl ether. The organic phases were combined, washed with saturated brine three times, dried over anhydrous sodium sulfate, and filtered. The filtrate was concentrated to give 34.9 g of a light-yellow solid. ¹H NMR (400 MHz, CDCl₃): δ 7.94 (dd, *J* = 8.0, 1.8 Hz, 1H), 7.82 (dd, *J* = 7.8, 1.8 Hz, 1H), 7.30 (t, *J=* 7.9 Hz, 1H), 3.95 (s, 3H), 2.67 (s, 3H).

### Step 2

The product from the previous step (32 g), DMF-DMA (39 g), and toluene (200 ml) were added into a 500 ml three-necked flask in sequence. After nitrogen gas replacement, the mixture was refluxed for 6 h at 110°C, and then concentrated under reduced pressure to remove most of the solvent. The concentrate was added with acetic acid (20.8 g), 80% hydrazine hydrate (16.4 g), and tetrahydrofuran (300 ml), heated to 65°C and stirred for reaction overnight. After the reaction, the reaction solution was concentrated to remove most of the solvent, added with ethyl acetate (1000 ml), washed three times with water (300 ml), dried over anhydrous sodium sulfate, and filtered. The filtrate was concentrated to remove most of the solvent, then added with petroleum ether (600 ml), triturated at room temperature for 30 minutes, filtered, and dried to obtain 32.5 g of a yellow solid. ¹H NMR (400 MHz, DMSO-*d₆*): δ 13.20 (s, 1H), 8.17 (d, *J=* 7.6 Hz, 1H), 7.90 (s, 1H), 7.83 (d, *J* = 7.7 Hz, 1H), 7.38 (t, *J* = 7.9 Hz, 1H), 6.77 (d, *J* = 2.1 Hz, 1H), 3.71 (s, 3H). MS: m/z 242.1 [M+Na]⁺.

### Step 3

The product from the previous step (21 g), diisopropylethylamine (18.6 g), DMAP (1.17 g), and dichloromethane (210 ml) were added into a 1000 ml three-necked flask in sequence. After the addition, the mixture was stirred for 10 minutes, then slowly dropwise added with a solution of SEM-Cl (38.4 g) in dichloromethane (50 ml), and reacted at room temperature after the addition. The system was added with saturated solution of ammonium chloride (400 ml) to quench the reaction, and then the resulting solution was separated. The organic phase was washed twice with NH₄Cl solution (400 ml), dried over anhydrous sodium sulfate, and filtered. The filtrate was concentrated and purified by silica gel column chromatography to obtain 38 g of a yellow oil (containing a small amount of SEM-Cl). MS: m/z 372.1 [M+Na]⁺.

### Step 4

The product from the previous step (38 g), 5% palladium on carbon (3.8 g) and ethanol (380 ml) were added into a 1000 ml single-necked flask. After the addition, the atmosphere in the flask was replaced with hydrogen gas sufficiently, and the mixture was reacted overnight at room temperature. After the reaction, the reaction solution was filtered, and the filtrate was concentrated under reduced pressure and then purified by silica gel column chromatography to obtain 22 g of a dark-red liquid. MS: m/z 320.2 [M+H]⁺.

### Step 5

The product from the previous step (22 g), 4,6-dichloro-*N-*(methyl-*d₃*)pyridazine-3-formamide (18.9 g), lithium chloride (8 g), and 2-methyltetrahydrofuran (400 ml) were added into a 3-liter three-necked flask. After the addition, the mixture was cooled down to 0°C, slowly dropwise added with LiHMDS (194 ml), naturally warmed up to room temperature after the addition, and reacted for 1 h. It was shown by TLC monitoring that a small portion of the raw materials were not fully reacted. The reaction system was added with saturated solution of ammonium chloride (400 ml) and extracted with ethyl acetate (400 ml). The organic phase was washed twice with saturated brine (300 ml), dried over anhydrous sodium sulfate, and filtered. The filtrate was added with triphenylchloromethane (19.2 g) and triethylamine (20 ml), and stirred at room temperature after the addition to remove the unreacted aniline compound. The reaction system was washed three times by adding saturated solution of ammonium chloride (400 ml). The organic phase was dried over anhydrous sodium sulfate and filtered. The filtrate was concentrated and purified by silica gel column chromatography to obtain 26.3 g of a yellow solid. MS: m/z 492.2 [M+H]⁺.

### Step 6

The product from the previous step (14.3 g), cyclopropanecarboxamide (4.76 g), BINAP (7.15 g), palladium acetate (0.66 g), cesium carbonate (47.6 g) and toluene (200 ml) were added into a 250 ml single-necked flask. After the addition, the atmosphere in the flask was replaced with nitrogen gas sufficiently, the mixture was heated to 90°C and reacted for 8 h with TLC monitoring. After the reaction, the reaction system was added with water (400 ml) and extracted with ethyl acetate three times. The organic phases were combined, washed once with saturated salt water (300 ml), dried over anhydrous sodium sulfate, and filtered. The filtrate was concentrated and purified by silica gel column chromatography to obtain 10.8 g of a light-yellow solid. MS: m/z 541.3 [M+H]⁺.

### Step 7

The product from the previous step (5.4 g) and tetraethylammonium fluoride trihydrate (25 g) were added into a 250 ml single-necked flask. The mixture was reacted at 85°C overnight with TLC monitoring. After the reaction, the reacting solution was added with a large amount of water, stirred for 30 minutes to precipitate white solid, and then filtered. The filter cake was dissolved in ethyl acetate (200 ml), and washed three times with water (200 ml). The organic phase was dried over anhydrous sodium sulfate, and filtered. The filtrate was concentrated, added with a mixed solvent of ethyl acetate and methanol (80:1) with an appropriate amount and triturated for 30 minutes. The resulting mixture was filtered again to obtain a white solid placed into a 50 ml single-necked flask. The white solid was triturated with 20 ml of ethyl acetate for 1 h, suction filtered, and dried to form 1.7 g of an off-white solid. MS: m/z 411.2 [M+H]⁺.

### Step 8

*N,N*-dimethylacetamide (15 ml), the product from the previous step (2.0 g), and 3-bromopropyne (5.2 g) were added into a 50 ml single-necked flask. The mixture was stirred until it was dissolved, then added with potassium carbonate (5.4 g) in batches, and reacted at room temperature for 24 h after the addition. After the reaction, the reaction solution was added with water (300 ml) and extracted with ethyl acetate three times. The organic phases were combined, washed three times with saturated brine (200 ml), dried over anhydrous sodium sulfate, and filtered. The filtrate was concentrated and purified by silica gel column chromatography to obtain 0.5 g of a light-yellow solid. ¹H NMR (400 MHz, DMSO-*d₆*): δ 11.34 (s, 1H), 11.00 (s, 1H), 9.16 (s, 1H), 8.18 (s, 1H), 7.90 (s, 1H), 7.69 (d, *J=* 7.5 Hz, 1H), 7.41 (d, *J* = 7.3 Hz, 1H), 7.24 (t, *J =* 7.7 Hz, 1H), 6.79 (s, 1H), 5.13 (s, 2H), 3.61 (s, 3H), 3.53 (s, 1H), 2.19-1.99 (m, 1H), 0.96-0.73 (m, 4H). MS: m/z 449.2126 [M+H]⁺.

### Example 14:

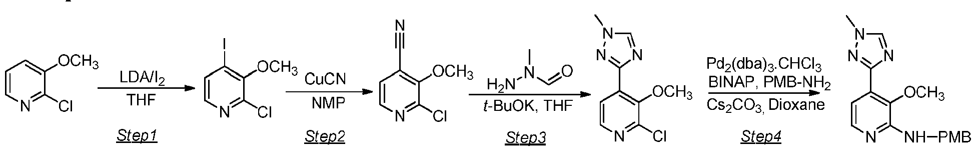

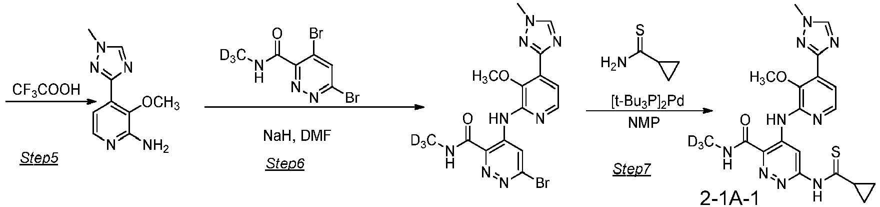

### Step 1

2-chloro-3-methoxypyridine (250.0 g) and tetrahydrofuran (2.5 L) were added into a reaction flask in sequence. Under nitrogen gas protection, the mixture was cooled down to -75±5°C and dropwise added with LDA (1.13 L) while the temperature was controlled at -75±5°C. After the addition, the resulting mixture was reacted for 2.5 to 3 hours at the maintained temperature of -75±5°C, then dropwise added with a solution of iodine (274.5g) in tetrahydrofuran (500 ml) while the temperature was controlled at -75±5°C. After the addition, the obtained mixture was naturally warmed up to 20 to 30°C and reacted for 2 to 3 hours. After the reaction, the reaction solution was dropwise added with saturated aqueous solution of ammonium chloride (4.0 L) and saturated aqueous solution of sodium thiosulfate (4.0 L) in sequence while the temperature was controlled at 20±5°C, stirred after the addition, and then extracted twice with n-hexane. The organic layers were combined, washed with saline solution, dried over anhydrous sodium sulfate, filtered, and concentrated under reduced pressure to obtain 420.0 g of a brown oil-solid mixture. The oil-solid mixture was subjected to trituration and drying to give 274.0 g of a yellow solid. MS: m/z 269.9, [M+H]⁺. ¹H NMR (400 MHz, CDCl₃): δ 7.78 (d, *J=* 5.0 Hz, 1H), 7.66 (d, *J=* 5.0 Hz, 1H), 3.91 (s, 3H).

### Step 2

2-chloro-4-iodo-3-methoxypyridine (100 g), *N*-methylpyrrolidone (500 ml), and cuprous cyanide (66.3 g, 0.74 mol) were added into a reaction flask in sequence. The mixture was heated to 120±5°C and stirred for 4-6 h. After the reaction, the reaction solution was cooled down to room temperature, and dropwise added with 500 ml of ammonia water while the temperature was controlled at 20±5°C. After the addition, the resulting solution was extracted with ethyl acetate. The organic layers were combined, washed with saline solution, dried over anhydrous sodium sulfate, filtered, and concentrated under reduced pressure to obtain 54.3 g of a pale brown solid, which then was purified by silica gel column chromatography to give 32.1 g of a white solid. MS: m/z 169.0, [M+H]⁺. ¹H NMR (400MHz, DMSO-*d₆*): δ 8.35 (d, *J=* 5.0 Hz, 1H), 7.90 (d, *J=* 4.9 Hz, 1H), 4.09 (s, 3H).

### Step 3

2-chloro-3-methoxycyanopyridine (3.0 g), methyl formylhydrazine (3.9 g), and tetrahydrofuran (80 ml) in sequence were added into a reaction flask. The mixture was cooled down to 0°C, and slowly dropwise added with a solution of potassium tert-butoxide (4.4 g) in tetrahydrofuran (50 ml) while the temperature was controlled at 0±5°C. After the addition, the resulting mixture was reacted for 0.5-1 h at the maintained temperature of 0±5°C. After the reaction, the reaction solution was poured into saturated aqueous solution of ammonium chloride (130 ml), and the obtained solution was extracted with ethyl acetate. The organic layers were combined, washed with saline solution, dried over anhydrous sodium sulfate, filtered, and concentrated under reduced pressure to obtain 3.5 g of a crude product, which then was purified by column chromatography to give 1.1 g of a white solid. MS: m/z 225.1, [M+H]⁺. ¹H NMR (400MHz, DMSO-*d₆*): δ 8.68 (s, 1H), 8.25 (d, *J=* 5.0 Hz, 1H), 7.90 (d, *J=* 5.0 Hz, 1H), 3.99 (s, 3H), 3.87 (s, 3H).

### Step 4

The product from the previous step (1.0 g), 4-methoxybenzylamine (10.8 g), tris(dibenzylideneacetone)dipalladium-chloroform adduct (0.46 g), 1.1'-binaphthyl-2.2'-diphemyl phosphine (1.1 g), cesium carbonate (4.35 g), and 1,4-dioxane (30 ml) in sequence were added into a reaction flask. Under nitrogen gas protection, the mixture was heated to 90±5°C and reacted for 14-16 h at the same temperature. After the reaction, the reaction solution was cooled down to 20-30°C, added with water, and extracted with ethyl acetate. The extracted solution was concentrated to obtain 4.5 g of an oily crude. MS: m/z 326.2 [M+H]⁺.

### Step 5

The crude product from the previous step (4.5 g) and trifluoroacetic acid (45 ml) were added into a reaction flask in sequence. The mixture was heated to 90±5°C and reacted for 1-2 h at the same temperature. After the reaction, the reaction solution was concentrated to dryness, added with methanol (110 ml) for dissolving, then added with sodium bicarbonate to adjust the pH to 7-8. The resulting solution was stirred for 30 minutes, filtered, and concentrated to obtain 2.51 g of a brown oil, which was then purified by silica gel column chromatography to obtain 2.1 g of a yellow oil-solid mixture. MS: m/z 206.1, [M+H]⁺. ¹H NMR (400MHz, DMSO-*d₆*): δ 8.62 (s, 1H), 7.73 (d, *J* = 5.6 Hz, 1H), 7.05 (d, *J* = 5.8 Hz, 1H), 6.77 (br s, 2H), 3.96 (s, 3H), 3.73(s, 3H).

### Step 6

3-methoxy-4-(1-methyl-1*H*-1,2,4-triazol-3-yl)pyridin-2-amine (1.2 g, 5.85 mmol), 4,6-dibromo-*N*-(methyl-*d₃*)pyridazin-3-formamide (2.27 g) and *N, N*-dimethylformamide (24 ml) in sequence were added into a reaction flask. Under nitrogen gas protection, the mixture was cooled down to 0±5°C, added with sodium hydride (0.94g), stirred at 0±5°C for 0.5-1 h, then naturally warmed up to 20±5°C and reacted for 3-4 h at the same temperature. After the reaction, the reaction solution was dropwise added into saturated aqueous solution of ammonium chloride while the temperature was controlled at 10±5°C. After the addition, the resulting solution was stirred for 1-2 h and filtered. The solid was washed twice with water, and dried to obtain 0.84 g of a pale brown solid. MS: m/z 424.1, [M+H]⁺. ¹H NMR (400MHz, DMSO-*d₆*): δ 12.56 (s, 1H), 9.48 (s, 1H), 9.37 (s, 1H), 8.68 (s, 1H), 8.22 (d, *J* = 5.3 Hz, 1H), 7.56 (d, *J* = 5.2 Hz, 1H), 3.99 (s, 3H), 3.91(s, 3H).

### Step 7

The product from the previous step (400 mg), N-methylpyrrolidone (16 ml), cyclopropanecarbothioamide (288.3 mg, 2.85 mmol), tricyclohexylphosphane (106.6 mg), N-methyldicyclohexylamine (556.7 mg), and bis(tri-tert-butylphosphine)palladium (97.5 mg) were added into a reaction flask in sequence. Under nitrogen gas protection, the mixture was heated to 110±5°C and reacted for 1-2 h at the same temperature. After the reaction, the reaction solution was cooled down to 20±5°C and dropwise added into a solution of glacial acetic acid. The obtained mixture was extracted three times with dichloromethane. The organic layers were combined, washed with saline solution, dried over anhydrous sodium sulfate, filtered, and concentrated under reduced pressure to obtain 1.2 g of a brown oil, which then was purified by silica gel column chromatography to give 30.0 mg of a yellow solid. MS m/z 443.2 [M+H]⁺.

### Example 15:

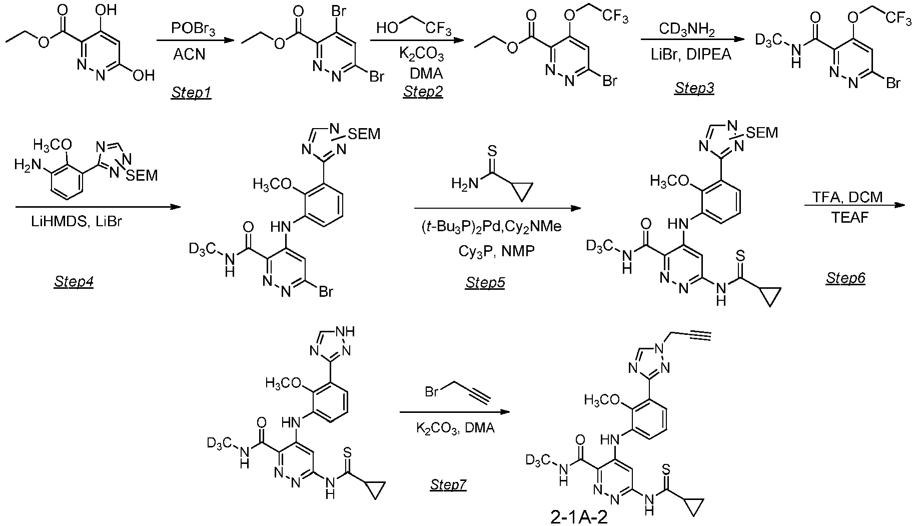

### Step 1

Ethyl-4,6-dihydroxypyridazine-3-carboxylate (8.5 g) and acetonitrile (100 ml) were added into a 250 ml single-necked flask. Under nitrogen gas protection, the mixture was cooled down to 0°C, and dropwise added with a solution of phosphorus oxybromide (39.7 g) in acetonitrile. After the addition, the resulting mixture was naturally warmed up to room temperature and stirred for reaction at 25°C, 55°C, 75°C, and 95°C for 40 minutes each, until the reaction was monitored by TLC to be completed. The system was added with 50 ml of ethyl acetate and filtered. The filtrate was concentrated to dryness, and the concentrate was added with 100 ml of dichloromethane for dissolving. The obtained solution was added with saturated solution of sodium bicarbonate to adjust the pH to 7-8, and then separated. The organic phase was dried over anhydrous sodium sulfate, filtered, concentrated, and purified by silica gel column chromatography to give 9.36 g of a yellow solid product.

### Step 2

The product from the previous step (7.3 g), DMA (75 ml), trifluoroethanol (2.61 g l), and potassium carbonate (4.9 g) were added into a 100 ml single-necked flask. After the addition, the mixture was stirred overnight at room temperature. The system was added with 200 ml of water and extracted with ethyl acetate. The organic phase was dried over anhydrous sodium sulfate, filtered, and concentrated under reduced pressure to dryness. The concentrate was triturated with a mixed solvent of methyl tert-butyl ether and n-hexane to obtain 4.86 g of a brown solid. ¹H NMR (400 MHz, CDCl₃): δ 7.29 (d, *J=* 6.9 Hz, 1H), 4.62-4.45 (m, 4H), 1.43 (t, *J=* 7.1 Hz, 3H). MS: m/z 329.0 [M+H]⁺.

### Step 3

The product from the previous step (4.86 g), acetonitrile (100 ml), and lithium bromide (5.15 g) were added into a 250 ml single-necked flask. Under nitrogen gas protection, the mixture was stirred at room temperature for 30 minutes, then cooled down to -26°C, added with diisopropylethylamine (9.58 g), stirred for 10 minutes, and added with deuterated methylamine hydrochloride (1.04 g) in batches at -30°C. After the addition, the reaction was continued at -25°C for 2 h until shown by TLC to be basically completed. The system was added with 200 ml of ice water and extracted with ethyl acetate. The organic phase was dried over anhydrous sodium sulfate, filtered, and concentrated under reduced pressure. The concentrate was triturated with petroleum ether to obtain 2.93 g of an off-white solid.

### Step 4

The product from the previous step (2.22 g), the aromatic amine compound (1.5 g), lithium bromide (814 mg), and 2-methyltetrahydrofuran (30 ml) were added into a 100 ml single-necked flask. Under nitrogen gas protection, the mixture was cooled down to 0°C, and dropwise added with LiHMDS (18.75 ml). After the addition, the obtained mixture was placed at room temperature and reacted for 3 h until shown by TLC to be basically completed, and then quenched by using 10% of ammonium chloride solution. The resulting solution was extracted with ethyl acetate, and the organic phase was dried over anhydrous sodium sulfate, filtered, concentrated under reduced pressure, and purified by silica gel column chromatography to obtain 2.1 g of a light yellow solid. ¹H NMR (400 MHz, DMSO-*d₆*): δ 11.10 (s, 1H), 9.38 (s, 1H), 8.83 (s, 1H), 7.74 (dd, *J* = 7.8, 4.1 Hz, 1H), 7.62 (dd, *J* = 7.9, 1.0 Hz, 1H), 7.34-7.31 (m, 2H), 5.58 (s, 2H), 3.71 (s,3H), 3.66 (t, *J* = 8.0 Hz, 2H), 0.87 (t, *J=* 8.0 Hz, 2H), -0.06 (s, 9H). MS: m/z 537.2 [M+H]⁺.

### Step 5

The product from the previous step (1.42 g), cyclopropanecarbothioamide (400 mg), tricyclohexylphosphane (222 mg), *N,N*-methyl dicyclohexylamine (1.55 g), bis(tri-tert-butylphosphine)palladium (200 mg), and *N*-methylpyrrolidone (14 ml) were added into a 100 ml single-necked flask. After nitrogen gas replacement, the mixture was heated to 110°C and reacted for 2 h. The system was added with water after cooling and extracted with ethyl acetate. The organic phase was dried over anhydrous sodium sulfate, filtered, concentrated under reduced pressure, and purified by silica gel column chromatography to obtain 1.0 g of a yellow oil. MS: m/z 558.1 [M+H]⁺.

### Step 6

The product from the previous step (900 mg), dichloromethane (16 ml), tetraethylammonium fluoride (2.7 g), and trifluoroacetic acid (40 ml) were added into a 100 ml single-necked flask. After the addition, the mixture was stirred and reacted for 2 h at room temperature. The system was concentrated under reduced pressure, and the concentrate was added with ethyl acetate for dissolving and washed with water three times. The organic phase was dried over anhydrous sodium sulfate, filtered, concentrated under reduced pressure, and purified by silica gel column chromatography to obtain 300 mg of a light-yellow solid. ¹H NMR (400 MHz, DMSO-*d₆*): δ 14.13 (s, 1H), 12.76 (s, 1H), 11.15 (s, 1H), 9.29 (s, 1H), 8.90 (s, 1H), 8.16 (brs, 1H), 7.74 (d, *J=* 6.7 Hz, 1H), 7.65 (d, *J=* 7.7 Hz, 1H), 7.32 (t, *J=* 7.6 Hz, 1H), 3.72 (s, 3H), 2.80-2.62 (m, 1H), 1.17-1.13 (m, 2H), 1.05-1.01 (m, 2H). MS: m/z 428.2 [M+H]⁺.

### Step 7

The product from the previous step (270 mg), DMA (5 ml), and bromopropyne (600 mg) were added into a 100 ml single-necked flask. After the addition, the mixture was heated to 50°C, added with potassium carbonate (610 mg) in batches, and then stirred for another 3 h. The reaction solution was added with water to quench the reaction, and then extracted with ethyl acetate. The organic phase was dried over anhydrous sodium sulfate, filtered, concentrated under reduced pressure, and purified by silica gel column chromatography to obtain 30 mg of a yellow solid. MS: m/z 466.2, [M+H]⁺.

### Example 16:

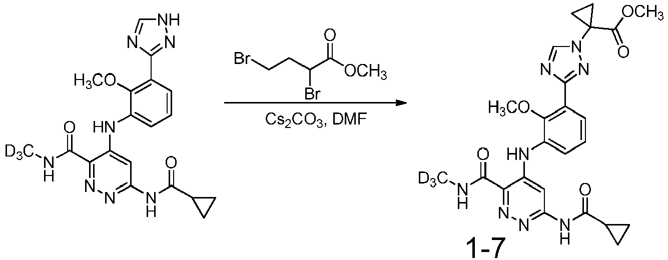

The triazole compound (100 mg), *N,N-*dimethylformamide (10 ml), methyl 2,4-dibromo butyrate (70 mg), and cesium carbonate (317 mg) were added into a 25 ml clean single-necked flask in sequence. After the addition, the atmosphere in the flask was replaced with nitrogen gas, and the mixture was reacted at 45°C until the reaction was monitored by TLC to be completed. The reaction solution was added into 50 ml of saturated aqueous solution of ammonium chloride, and the resulting solution was extracted with ethyl acetate (30 ml). The organic phases were combined, washed with saturated brine (30 ml), dried over anhydrous sodium sulfate, filtered, and concentrated under reduced pressure to obtain about 200 mg of a crude product, which then was purified by silica gel column chromatography to give 80 mg of a product. ¹H NMR (400 MHz, CDCl₃): δ 11.04 (s, 1H), 9.63 (s, 1H), 8.30 (s, 1H), 8.25 (s, 1H), 8.07 (s, 1H), 7.80 (dd, *J=* 7.8, 1.5 Hz, 1H), 7.55 (dd, *J* = 8.0, 1.5 Hz, 1H), 7.28 (t, *J* = 7.9 Hz, 1H), 3.81 (s, 3H), 3.73 (s, 3H), 1.99-1.92 (m, 2H), 1.87-1.78 (m, 1H), 1.78-1.71 (m, 2H), 1.16-1.08 (m, 2H), 0.95-0.87 (m, 2H). MS: m/z 510.2 [M+H]⁺.

### Example 17:

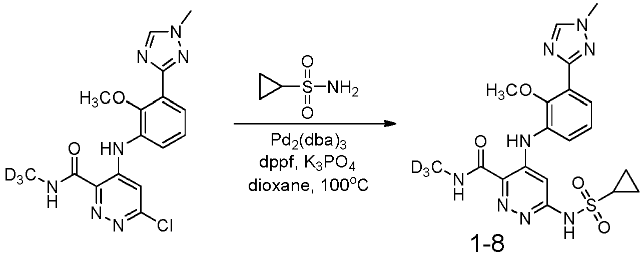

6-chloro-4-((2-methoxy-3-(1-methyl-1*H*-1,2,4-triazol-3-yl)phenyl)amino)-*N-*(methyl-*d₃*)pyr idazine-3-formamide (500 mg, 1.33 mmol), cyclopropanesulfonamide (322 mg), potassium phosphate (560 mg), 1,1'-bis(diphenylphosphino)ferrocene (150 mg), tris(dibenzylideneacetone)dipalladium (119 mg), and 1,4-dioxane (15 ml) were added into a 100 ml single-necked flask in sequence. After the addition, the atmosphere in the flask was replaced with nitrogen gas sufficiently, and the mixture was stirred at 100°C for 24 h. The reaction solution was concentrated to dryness, added with 20 ml of water, and extracted with dichloromethane. The organic layers were combined, dried over anhydrous sodium sulfate, concentrated to dryness, and purified by silica gel column chromatography to obtain 330 mg of a yellow solid. ¹H NMR (400 MHz, CDCl₃): δ 12.61 (br s, 1H), 11.08 (s, 1H), 8.15 (s, 1H), 7.88 (dd, *J* = 7.8, 1.6 Hz, 1H), 7.66 (s, 1H), 7.45 (dd, *J* = 8.0, 1.5 Hz, 1H), 7.27 (t, *J* = 7.9 Hz, 1H), 4.03 (s, 3H), 3.82 (s, 3H), 2.65-2.52 (m, 1H), 1.22-1.13 (m, 2H), 0.99-0.90 (m, 2H). ¹³C NMR (100 MHz, CDCl₃): δ 164.7, 160.0, 155.9, 151.8, 146.9, 144.0, 131.3, 131.0, 128.2, 126.2, 124.6, 124.3, 98.5, 61.8, 36.5, 31.7, 5.47. MS: m/z 462.2, [M+H]⁺.

### Example 18:

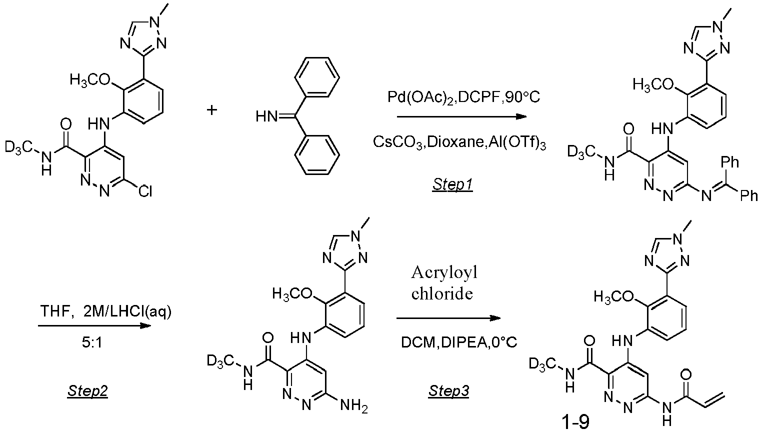

### Step 1

The raw material (1 g), benzophenonimine (3.0 g), palladium acetate (185 mg), cesium carbonate (13.4 g), 1,1'-di(dicyclohexylphosphino)-ferrocene (1.9 g), aluminum trifluoromethanesulfonate (195 mg), and dioxane (120 ml) were added into a 250 ml single-necked flask. Under the protection of replaced nitrogen gas, the mixture was heated to 100°C and reacted for 10 h. The system was cooled down, poured into 300 ml of water for washing, extracted with 300 ml of ethyl acetate, and separated. The separated solution was subjected to concentration and column chromatography to yield 2 g of a light-yellow solid product. MS: m/z 522.24, [M+H]⁺.

### Step 2

The raw material (2.2 g) and THF (80 ml) were added into a 100 ml single-necked flask. The mixture was slowly added with 2 M/L Hclaq (16ml) at room temperature, stirred and reacted at the same temperature for 2 h after the addition. The system was poured into 200 ml of saturated solution of sodium bicarbonate for washing, extracted with 200 ml of ethyl acetate, and separated. The separated solution was subjected to drying, concentration and column chromatography to obtain 1.1 g of a reddish-brown solid product. MS: m/z 358.18, [M+H]⁺.

### Step 3

The raw material (500 mg), dichloromethane (80 ml) used for dissolving, and DIPEA (180 mg) were added into a 250 ml single-necked flask. The mixture was cooled down to 0°C by ice bath, dropwise added with 20 ml of a solution of acryloyl chloride (130 mg) in dichloromethane, and stirred at room temperature for reaction after the addition. The system was washed with 100 ml of water and separated. The separated solution was subjected to drying, concentration and column chromatography to obtain 150 mg of a yellow solid product. ¹H NMR (400 MHz, CDCl₃): δ 11.26 (s, 1H), 11.01 (s, 1H), 9.17 (s, 1H), 8.57 (s, 1H), 8.28 (s, 1H), 7.69 (dd, *J* = 1.5, 7.8 Hz, 1H), 7.58 (dd, *J =* 1.4, 7.9 Hz, 1H), 7.32 (t, *J* = 7.9 Hz, 1H), 6.69-6.62 (m, 1H), 6.33 (dd, *J* = 1.7, 17.0 Hz, 1H), 5.84 (dd, *J* = 1.6, 10.1 Hz, 1H), 3.95 (s, 3H), 3.73 (s, 3H); MS: m/z 412.19, [M+H]⁺.

### Example 19:

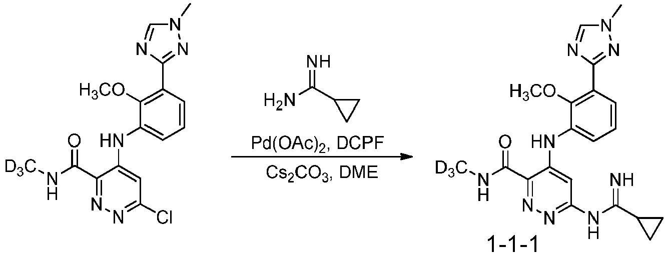

6-chloro-4-((2-methoxy-3-(1-methyl-1*H*-1,2,4-triazol-3-yl)phenyl)amino)-*N*-(methyl-*d₃*)pyr idazine-3-formamide (1.5 g), cesium carbonate (5.2 g), cyclopropanecarboxamidine hydrochloride (962 mg), palladium acetate (89 mg), 1,1'-di(dicyclohexylphosphino)-ferrocene (884 mg), and ethylene glycol dimethyl ether (100 ml) were added into a 250 ml single-necked flask. After nitrogen gas replacement, the mixture was heated to 90°C and reacted. After cooling, the reaction solution was poured into 200 ml of water, and extracted with ethyl acetate. The extractd solution was dried over anhydrous sodium sulfate, filtered, concentrated under reduced pressure and purified by silica gel column chromatography to obtain 800 mg of a light yellow solid. ¹H NMR (400 MHz, DMSO-*d₆*): δ = 10.70 (s, 1H), 9.44 (br s, 1H), 9.12 (s, 1H), 8.57 (s,1H), 8.28 (br s, 1H), 7.64 (dd, *J* = 7.8, 1.4 Hz, 1H), 7.51 (d, *J* = 7.9 Hz, 1H), 7.27 (t, *J* = 7.9 Hz, 1H), 6.50 (s, 1H), 3.95 (s, 3H), 3.72 (s, 3H), 1.69-1.57 (m, 1H), 0.98-0.89 (m, 2H), 0.83-0.74 (m, 2H). ¹³C NMR (100 MHz, DMSO-*d₆*): δ = 167.4, 167.0, 166.5, 159.4, 150.9, 145.6, 144.9, 134.3, 133.3, 126.7, 126.2, 124.8, 123.7, 102.9, 61.6, 36.5, 16.0, 8.4. MS: m/z 425.2, [M+H]⁺.

### Example 20:

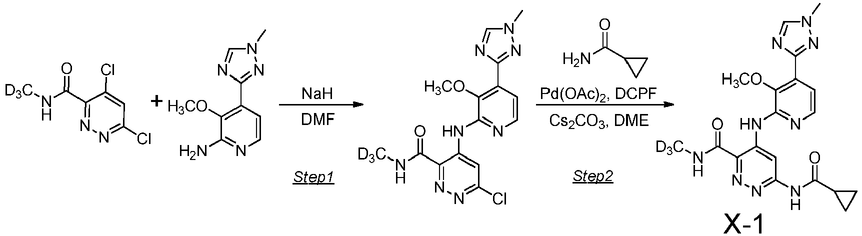

### Step 1

3-methoxy-4-(1-methyl-1*H*-1,2,4-triazol-3-yl)pyridin-2-amine (1.4 g) and *N,N*-dimethylformamide (50 ml) were added into a reaction flask. The mixture was cooled down to 0°C, added with sodium hydride (1.1 g) in batches, stirred for 20 minutes after the addition, then slowly warmed up to room temperature and stirred for 30 minutes. The reaction solution was cooled down to 0°C, slowly dropwise added with a tetrahydrofuran solution (40 ml) of 4,6-dichloro-*N*-(methyl-*d*₃)pyridazine-3-formamide (4.3 g) while the temperature was controlled to be less than 5°C. The addition was completed within 3 h, and the stirring was carried out overnight. The reaction solution was added with aqueous solution of ammonium chloride (20 ml) and water (40 ml) to, stirred for 30 minutes, and then filtered. The filter cake was washed twice with water, then dried to give 1.82 g of a gray-white solid. MS: m/z 378.1 [M+H]⁺.

### Step 2

The product from the previous step (1.82 g, 4.8 mmol), DCPF (1.1 g), palladium acetate (108 mg), cesium carbonate (7.9 g), and DME (60 ml) were added into a reaction flask in sequence. After nitrogen gas replacement, the mixture was heated to 90°C and reacted for 1 h. The reaction solution was cooled down to room temperature and filtered. The filtrate was concentrated and then purified by silica gel column chromatography to obtain 430 mg of a light yellow solid compound. ¹H NMR (400 MHz, DMSO-*d₆*): δ 12.44 (s, 1H), 11.36 (s, 1H), 9.89 (s, 1H), 9.25 (s, 1H), 8.67 (s, 1H), 8.14 (d, *J* = 5.2 Hz, 1H), 7.50 (d, *J* = 5.2 Hz, 1H), 4.00 (s, 3H), 3.91 (s, 3H), 2.20-2.09 (m, 1H), 0.96-0.81 (m, 4H). ¹³C NMR (100 MHz, DMSO-*d₆*): δ 173.8, 167.0, 157.6, 156.6, 150.1, 146.2, 142.6, 142.5, 142.0, 135.5, 131.4, 117.2, 102.2, 61.7, 36.8, 14.9, 8.6. MS: m/z 427.2 [M+H]⁺.

### Example 21:

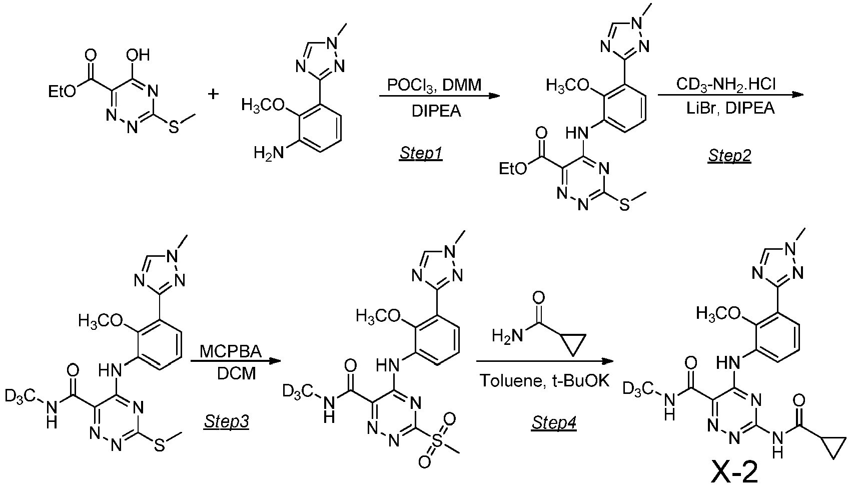

### Step 1

Ethyl 5-hydroxy-3-methylthio-1,2,4-triazine-6-formate (4.62 g) and acetonitrile (50 ml) were added into a reaction flask. The mixture was cooled down to 0°C, added with DIPEA (5.17 g) and N-methylmorpholine (101 mg), then slowly dropwise added with phosphorus oxychloride (4.6 g), slowly warmed up to room temperature after the addition, and reacted for 1 h. After the reaction completed, the reaction solution was cooled down to 0°C, added with DIPEA to adjust the pH to about 7-8, then dropwise added with a tetrahydrofuran solution (10 ml) of 2-methoxy-3-(1-methyl-1*H*-1,2,4-triazol-3-yl)aniline (3.27 g). After the addition, the solution was warmed up to 30°C and reacted for 4 h. The system was added with saturated aqueous solution of ammonium chloride to for washing, and extracted with dichloromethane. The organic phase was dried, concentrated, and purified by silica gel column chromatography to obtain 5.0 g of a product.

### Step 2

The product from the previous step (5.0 g), deuterated methylamine hydrochloride (1.02 g), and acetonitrile (50 ml) were added into a reaction flask. The mixture was cooled down to 0°C, added with DIPEA (4.65 g) and lithium bromide (2.08 g), slowly warmed up to room temperature after the addition, and reacted for 1 h. The system was added with saturated aqueous solution of ammonium chloride and extracted with dichloromethane. The organic phase was dried, concentrated, and purified by silica gel column chromatography to obtain 4.18 g of a product.

### Step 3

The methyl sulfide compound (600 mg) and dichloromethane (20 ml) were added into a 50 ml single-necked flask. Under the protection of replaced nitrogen gas, the mixture was added with MCPBA (800 mg) in batches, stirred and reacted at room temperature for 3 h. The system was poured into aqueous solution of sodium bicarbonate, then added with 1 g of sodium thiosulfate, stirred for 10 minutes, and extracted with dichloromethane. The organic phase was dried over anhydrous sodium sulfate, filtered, concentrated, and purified by silica gel column chromatography to obtain 200 mg of a yellow solid. MS: m/z 422.1, [M+H]⁺.

### Step 4

The product from the previous step (100 mg), cyclopropanecarboxamide (50 mg), potassium tert-butoxide (150 mg), and toluene (10 ml) were added into a 50 ml single-necked flask. Under nitrogen gas protection, the mixture was heated to 80°C and reacted for 3 h. The system was poured into aqueous solution of ammonium chloride and extracted with ethyl acetate. The organic phase was dried over anhydrous sodium sulfate, filtered, concentrated under reduced pressure, and purified by silica gel column chromatography to obtain 30 mg of a yellow solid. ¹H NMR (400 MHz, CDCl₃): δ 12.10 (s, 1H), 8.85 (dd, *J* = 8.2, 1.2 Hz, 1H), 8.71 (s, 1H), 8.14 (s, 1H), 7.92 (s, 1H), 7.78 (dd, *J* = 7.9, 1.5 Hz, 1H), 7.31 (t, *J* = 8.1 Hz, 1H), 4.04 (s, 3H), 3.93 (s, 3H), 2.37-2.27 (m, 1H), 1.26-1.21 (m, 2H), 1.01-0.95 (m, 2H). MS: m/z 427.2 [M+H]⁺.

### Example 22:

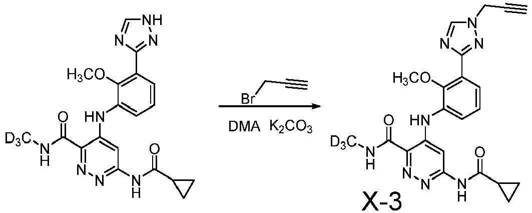

6-(cyclopropylamido)-4-((2-methoxy-3-(1*H*-1,2,4-triazol-3-yl)phenyl)amino)-*N*-(methyl-*d₃* )pyridazine-3-formamide (500 mg, 1.2 mmol), *N*,*N*-dimethylacetamide (20 ml), propargyl bromide (1.3 g, 10.9 mmol), and potassium carbonate (1.3 g, 9.4 mmol) were added into a 50 ml single-necked flask. After the addition, the mixture was reacted for 2 h at room temperature. After the reaction completed, the reaction solution was poured into 400 ml of water for quenching, and extracted twice with ethyl acetate. The organic phases were combined, dried over anhydrous sodium sulfate, filtered, concentrated under reduced pressure, and purified by silica gel column chromatography to obtain 250 mg of an off-white solid. ¹H NMR (400 MHz, CDCl₃): δ 11.01 (s, 1H), 9.81 (s, 1H), 8.39 (s, 1H), 8.25 (s, 1H), 8.06 (s, 1H), 7.82 (dd, *J* = 7.9, 1.6 Hz, 1H), 7.55 (dd, *J* = 8.0, 1.5 Hz, 1H), 7.28 (t, *J* = 7.9 Hz, 1H), 5.08 (d, *J* = 2.5 Hz, 2H), 3.83 (s, 3H), 2.64 (t, *J =* 2.6 Hz, 1H), 1.91-1.82 (m, 1H), 1.15-1.08 (m, 2H), 0.94-0.87 (m, 2H). ¹³C NMR (100 MHz, CDCl₃): δ 173.2, 166.7, 160.6, 155.7, 151.5, 146.1, 143.0, 135.0, 132.4, 127.1, 125.7, 124.6, 123.6, 97.8, 76.1, 75.1, 61.6, 39.7, 16.0, 8.8, MS: m/z 450.2102 [M+H]⁺.

### Biological test and other application examples

### Biological test example 1: Efficacy test in psoriasis-like mouse model

**Experimental animal:** Balb/c mice.

### Animal grouping:

(1) negative control group (applying Vaseline, administering solvent intragastrically),
(2) model control group [applying imiquimod (IMQ)],
(3) administration group (applying IMQ, administering Compound 7, Compound 8, Compound 9, Compound 10, and Compound No. 146 intragastrically);
wherein, "Compound No. 146" refers to the compound in Example 146 of reference WO2014074661, which was prepared according to the method described therein.

**Administration dosage and method:** 20 mg/kg, BID, intragastric administration.

**Experiment process:** On Day 0, the animals were subjected to hair shaving on the test area of the back, and then they were grouped. From Day 1 to Day 7, one hour after the first administration in every morning, 40 mg of 5% imiquimod cream was applied once to the test area on the back, after that, a second administration was conducted on the same day. The skin lesion symptoms of psoriasis and efficacy were evaluated through Psoriasis Area and Severity Index (PASI) score (erythema, scale, thickening degree). The higher the score, the more severe the symptoms are.
PASI score results on day 7 are listed in the table below, wherein,
"+" represents a score of ≥ 6;
"++" represents a score of greater than 5 and less than 6;
"+++" represents a score of greater than 4 and less than or equal to 5;
"++++" represents a score of less than or equal to 4.

| Administration group | PASI score |
|---|---|
| Model control group | + |
| Compound No. 146 | ++ |
| Compound 7 | ++++ |
| Compound 8 | ++++ |
| Compound 9 | ++++ |
| Compound 10 | ++++ |

The above data indicate that Compound 7, Compound 8, Compound 9, and Compound 10 can improve psoriasis symptoms in psoriasis-like mouse model induced by imiquimod, while Compound No. 146 can also improve psoriasis symptoms in psoriasis-like mouse model induced by imiquimod. Compound 7, Compound 8, Compound 9, and Compound 10 have better effects than Compound No. 146.

### Biological test example 2: Efficacy test in psoriasis-like mouse model

By using the same scheme as test example 1, pharmaceutical efficacy in psoriasis-like mouse model induced by imiquimod was studied. The data indicate that Compound 11 can improve psoriasis symptoms in psoriasis-like mouse model induced by imiquimod, and Compound 11 has a better effect than Compound No. 146.

Wherein, "Compound No. 146" refers to the compound in Example 146 of reference WO2014074661, which was prepared according to the the method therein.
PASI score results on day 7 are listed in the table below, wherein,
"+" represents a score of ≥ 6;
"++" represents a score of greater than 5 and less than 6;
"+++" represents a score of greater than 4 and less than or equal to 5;
"++++" represents a score of less than or equal to 4.

| Administration group | PASI score |
|---|---|
| Model control group | + |
| Compound No. 146 | ++ |
| Compound 11 | ++++ |

### Biological test example 3: Enzymology experiment in vitro

**Experiment steps:** The compound was dissolved in DMSO to a storage concentration of 10 mM. The compound with a gradient of different concentrations which was 200 times of the final concentration was prepared in a compound dilution plate, and then transferred to an Echo plate. 75 nL of the compound was transferred from the Echo plate to a 384-well experimental plate by using an Echo instrument. 5 µl of TYK2-JH2 kinase with a concentration that was 3 times of the final concentration was added into the 384-well plate. 5 µl of Tb antibody with a concentration that was 3 times of the final concentration was added into the 384-well plate. 5 µl of Tracer with a concentration that was 3 times of the final concentration was added into the 384-well plate. The plate was centrifuged for 30 seconds and incubated at room temperature for 60 minutes. The fluorescence signal ratio value of 495 nm/520 nm was read by a Envision microplate reader (PerkinElmer). Data analysis was performed by using XL-Fit software and IC50 of the compound was calculated.
Wherein, "A" represents that TYK2-JH2 binding inhibitory activity (IC50 value) is less than 0.10 nM;
"B" represents that TYK2-JH2 binding inhibitory activity (IC50 value) is ranging from 0.10 nM to 0.50 nM;
"C" represents that TYK2-JH2 binding inhibitory activity (IC50 value) is ranging from 0.50 nM to 1 nM;
"D" represents that TYK2-JH2 binding inhibitory activity (IC50 value) is greater than 1 nM.

| Compound number | TYK2-JH2 binding inhibitory activity (IC50 value) |
|---|---|
| Compound 7 | A |
| Compound 8 | A |
| Compound 9 | A |
| Compound 2-1A-1 | A |
| Compound 11 | A |
| Compound 2-1B-1 | B |
| Compound 2-1B-2 | C |
| Compound 2-1B-7 | B |
| Compound X-1 | B |

The above data indicate that Compound 7, Compound 8, Compound 9, Compound 10, Compound 11, Compound 2-1B-1, Compound 2-1B-2, Compound 2-1B-7, and Compound X-1 all have inhibitory effects on the activity of TYK2-JH2, wherein part of the compounds have significant effects.

### Biological test example 4: Inhibitory effect test of compounds on pSTATS expression in CD3+ cells using the technology of fluorescence activated cell sorting (FACS)

Compound dilution and preparation: The test compound was prepared into a 10 mM solution using DMSO, and diluted in DMSO to obtain a solution with a gradient of different concentrations which was 500 times of the final concentration. 5 µL of the diluted compound was transferred into 120 µL of PBS solution containing 0.1% BSA. Positive and negative control groups were set up with 0.2% DMSO ultimately contained in each group.

Experiment steps:
1) Human PBMC cells were added into a 96-well cell culture plate, with 0.5 million cells and a volume of 67.5 µL per well.
2) 3.5 µL of the diluted compound was added and mixed evenly.
3) The incubation was carried out in a 37°C incubator for 60 minutes.
4) IFN-alpha was diluted to 600 ng/mL in DPBS containing 0.1% BSA. After the above 60-minute incubation, 5 µL of PE-anti-hCD3 antibody was added into each well, then 4 µL of the diluted IFN-alpha was also added into each well.
5) The incubation was carried out in a 37°C incubator for 30 minutes.
6) All cells were transferred into a 96-deep-well plate and added with 1 mL of Lyse/fix buffer preheated at 37°C.
7) The incubation was carried out in dark at 37°C for 10 minutes.
8) The plate was centrifuged at 600 g for 5 minutes with the supernatant abandoned, then washed twice with 1 mL of PBS and centrifuged.
9) 1 mL of Perm buffer III was added into the cell precipitation.
10) The incubation was carried out in dark at 4°C for 30 minutes.
11) The plate was centrifuged at 600 g for 5 minutes with the supernatant abandoned, then washed twice with 1 mL of PBS and centrifuged.
12) APC anti-human pSTAT5 antibody was diluted 200 times in Staining buffer, then added into the cell wells with 100 uL per well, and mixed evenly.
13) The incubation was carried out at room temperature for 40 minutes.
14) The plate was washed twice with 1 mL of Staining buffer per well, and centrifuged at 600 g for 5 minutes.
15) After the supernatant was abandoned, the cell precipitation was resuspended in 300 uL of Staining buffer.
16) The sample was loaded and analyzed in a Beckman CytoFlex flow cytometer.

The experiment results are summarized as follows:
wherein, "A" represents an inhibitory activity on p-STAT5 expression (IC50 value) less than 1 nM;
"B" represents an inhibitory activity on p-STAT5 expression (IC50 value) ranging from 1 nM to 5 nM;
"C" represents an inhibitory activity on p-STAT5 expression (IC50 value) ranging from 5 nM to 10 nM;
"D" represents an inhibitory activity on p-STAT5 expression (IC50 value) greater than 10 nM.

| Compound number | Inhibitory activity on p-STAT5 expression (IC50 value) |
|---|---|
| Compound 7 | A |
| Compound 8 | A |
| Compound 9 | A |
| Compound 10 | A |
| Compound 11 | A |
| Compound 2-1A-1 | A |
| Compound 2-1B-1 | C |
| Compound 2-1B-2 | C |
| Compound 2-1B-7 | C |
| Compound X-1 | B |

The above data indicate that Compound 7, Compound 8, Compound 9, Compound 10, Compound 11, Compound 2-1A-1, Compound 2-1B-1, Compound 2-1B-2, Compound 2-1B-7, and Compound X-1 have inhibitory effects on p-STAT5 expression; wherein part of the compounds have significant inhibitory activities.

### Biological test example 5: Pharmacokinetic study in vivo

**Experimental animal:** 8 to 10 week-old male SD rats, fasted 12 hours before administration.

### Formulation method

**Intragastric administration:** mixed solution (ethanol+TPGS+PEG300=5:5:90) was used as the administration solvent.

**Injection administration:** mixed solution (PEG400+ddH20=80:20) was used as the administration solvent.

**Administration capacity:** 5 ml/kg.

**Animal grouping:** intravenous IV group and intragastric PO group, with 3 male SD rats in each group.

**Administration route and dosage:** 1 mg/kg for intravenous IV group; 10 mg/kg for intragastric PO group.

**Administration frequency:** single administration.

### Sampling:

IV blood collection points: 5 minutes, 15 minutes, 30 minutes, 1 hour, 2 hours, 4 hours, 6 hours, 8 hours, and 24 hours after administration;
PO blood collection points: 15 minutes, 30 minutes, 1 hour, 2 hours, 4 hours, 6 hours, 8 hours, and 24 hours after administration.

### Sample treatment

The whole blood of rat was centrifuged at 10000 rpm for 5 minutes to separate plasma, which was stored at 4°C. 0.1 ml of the plasma was taken, added with 0.2 ml of acetonitrile, vortexed for 1 minute, and centrifuged at 10000 rpm for 5 minutes to take the supernatant, which was sent for analysis of drug content after 0.22 µm filtration.

The test results are summarized as follows:

| Compound number | Absolute bioavailability(F%) |
|---|---|
| Compound 11 | 97.8% |
| Compound No. 146 | 73.3% |

wherein, "Compound No. 146" refers to the compound in Example 146 of reference WO2014074661, which was prepared according to the method described in the reference.

The results of the pharmacokinetic study *in vivo* on Compound 11 and Compound X-1 show that the absolute bioavailability of Compound 11 is higher than that of Compound No. 146.

### Biological test example 6: Efficacy test in psoriasis-like mouse model induced by imiquimod

By using the same scheme as test example 1, pharmaceutical efficacy in psoriasis-like mouse model induced by imiquimod was studied. PASI score results on Day 7 are listed in the table below, wherein,
"+" represents a score of ≥ 6;
"++" represents a score of greater than 5 and less than 6;
"+++" represents a score of greater than 4 and less than or equal to 5;
"++++" represents a score of less than or equal to 4.

| Administration group | PASI score |
|---|---|
| Model control group | + |
| Compound No. 146 | ++ |
| Compound X-1 | ++ |
| Compound 1-1 | ++ |
| Compound 1-2 | ++ |
| Compound 1-3 | ++ |
| Compound 1-4 | ++ |
| Compound 1-5 | ++ |
| Compound 1-6 | ++ |
| Compound 1-7 | ++ |
| Compound 1-8 | + |
| Compound 1-9 | + |
| Compound 1-1-1 | + |
| Compound 2-1A-1 | ++++ |
| Compound 2-1B-1 | ++ |
| Compound 2-1B-2 | ++ |
| Compound 2-1B-7 | ++ |
| Compound 7 | ++++ |
| Compound 8 | ++++ |
| Compound 9 | ++++ |
| Compound 11 | ++++ |

The above data indicate that the listed compounds can improve psoriasis symptoms in psoriasis-like mouse model induced by imiquimod, wherein part of them have significant effects.

### Application example 7: Preparation of ointment

The acting pathway of the compounds of the present invention involves autoimmune diseases and other diseases, such as ankylosing spondylitis, atopic dermatitis, enteritis, etc. Some diseases involve skin related diseases. Under certain circumstances, it is more convenient and immediate for use to develop the compounds of the present invention as external preparations.

### Composition of ointment formulation:

| **Name** | **Proportion in the formulation** |
|---|---|
| Compound 7 or Compound X-1 | 5% |
| Vaseline | 70% |
| Light liquid paraffin | 23.5% |
| Antioxidant (BHA) | 1.0% |
| Antibacterial agent (benzyl alcohol) | 0.5% |
| Total | 100% |

### Preparation of ointment:

**Homogenization treatment:** Vaseline in the formulation amount was put into the main pot, heated at 70°C to 90°C until completely melted, with the temperature controlled at 75±5°C for use. Compound 7 or Compound X-1, antioxidant BHA (butyl hydroxyanisole), and benzyl alcohol were added into about half of light liquid paraffin and evenly dispersed with mechanical stirring to obtain Mixture 1. Mixture 1, the remaining light liquid paraffin, and the melted Vaseline solution were added into the main pot, subjected to scraped-surface stirring at 30 to 60 rpm and then subjected to homogenization for 20 to 30 minutes at a stirring speed of 1000 to 2800 rpm, a vacuum of -50 to -100KPa and a temperature of 70±5°C. After that, the resulting mixture was slowly cooled down to a temperature below 35°C to obtain a paste.

**Filling:** The obtained paste was subjected to filling by using a filling machine according to established specification, during which the jacket temperature of the charging hopper was controlled at 25 to 30°C, and the sealing temperature was controlled at 450±20°C. According to the above formulation and ointment preparation protocol, the ointment of Compound 7 and the ointment of Compound X-1 were obtained.

### Summary of long-term stability observation results of the ointments:

| **Ointment** | **Observation at room temperature 10-day observation** | **Observation at room temperature 20-day observation** | **Observation at room temperature 30-day observation** |
|---|---|---|---|
| 5% ointment (Compound 7) | Uniform paste | Uniform paste | Uniform paste |
| 5% ointment (Compound X-1) | Uniform paste | Visible granular substance appeared in the paste | Visible granular substance appeared in the paste |

Comparing the stability of the 5% ointment of Compound 7 with that of the 5% ointment of Compound X-1, it can be seen that after 20 days storage, visible granular substance appeared in the paste of the ointment comprising Compound X-1 at a content of 5%, which may arise from precipitation of Compound X-1 after the long-term storage. However, for the ointment comprising Compound 7 at a content of 5%, the paste remained uniform without any visible granular substance after 30 days storage. The above results indicate that the ointment prepared from Compound 7 at a content of 5% is beneficial for storage and use.

### Biological test example 8: Efficacy test in rat model with increased IL-17 induced by imiquimod using intragastric administration method

Diseases regulated by IL-17 include autoimmune diseases such as ankylosing spondylitis, rheumatoid arthritis, atopic dermatitis, enteritis, etc. The compounds of the present invention have potential therapeutic effects on autoimmune diseases by regulating IL-17 level.

**Experimental animal:** 8 to 10-week old SD rats, half male and half female.

### Experimental grouping:

(1) negative control group (applying Vaseline, administering solvent intragastrically),
(2) model control group [applying imiquimod (IMQ)],
(3) administration group (applying IMQ, administering the compounds of the present invention intragastrically).

**Administration dosage and method:** 20 mg/kg, BID, intragastric administration.

**Model establishment:** SD rat was subjected to application of 40 mg of imiquimod ointment on its back skin once a day to construct the rat model.

**Experiment test:** On day 6, the content of IL-17F cell factor in blood was measured.

### Test results:

A represents an IL-17F content less than 30 pg/mL;
B represents an IL-17F content ranging from 30 pg/mL to 100 pg/mL;
C represents an IL-17F content ranging from 100 pg/mL to 300 pg/mL;
D represents an IL-17F content ranging from 300 pg/mL to 400 pg/mL;
E represents an IL-17F content greater than 400 pg/mL.

| Experiment group | Content level of IL-17F in skin |
|---|---|
| negative control group | A |
| model control group | E |
| Compound 7 treatment group | A |
| Compound 11 treatment group | A |
| Compound X-1 treatment group | C |

The study on the effect of different compounds on imiquimod induction rat model showed that Compound 7 has better effect than Compound X-1. After the treatment with Compound 7 or Compound 11 by intragastric administration, the level of inflammatory factor IL-17F in the experimental animals was close to that of normal animals. At the same time, it was also observed that after the treatment with Compound 7 or Compound 11 by intragastric administration, the skin lesions in rats were significantly reduced, and the area and severity index score of skin lesions in rats were superior to that of Compound X-1 treatment group. Further experiments show that they are also superior to other compounds with similar skeletons.

### Biological test example 9: Efficacy test in rat model with increased IL-17 induced by imiquimod using intragastric administration method

By using the same scheme as biological test example 8, the efficacy of the compounds of the present invention was evaluated, and the following test results were obtained:

### Test results:

A represents an IL-17F content less than 30 pg/mL;
B represents an IL-17F content ranging from 30 pg/mL to 100 pg/mL;
C represents an IL-17F content ranging from 100 pg/mL to 300 pg/mL;
D represents an IL-17F content ranging from 300 pg/mL to 400 pg/mL;
E represents an IL-17F content greater than 400 pg/mL.

| Experiment group | Content level of IL-17F in skin |
|---|---|
| Negative control group | A |
| Model control group | E |
| Compound 2-1A-1 treatment group | A |
| Compound 1-1-1 treatment group | D |
| Compound X-1 treatment group | C |

Study on imiquimod induced rat model treated with different compounds showed that Compound 2-1A-1 has better therapeutic effect than Compound 1-1-1 and Compound X-1. After the treatment with Compound 2-1A-1 by intragastric administration, the level of inflammatory factor IL-17F in the experimental animals was close to that of normal animals. At the same time, it was also observed that after the treatment with Compound 2-1A-1 by intragastric administration, the skin lesions in rats were significantly reduced, and the area and severity index score of skin lesions in rats were superior to that of Compound 1-1-1 and Compound X-1 treatment groups.

### Biological test example 10: Efficacy test in rat model with increased IL-17 induced by imiquimod using application method

**Experimental animal:** 8 to 10 week-old SD rats, half male and half female.

**Model establishment:** The SD rat was subjected to application of 40 mg of imiquimod ointment on its back skin once a day to construct psoriasis-like rat model.

**Experiment process:** Hair removal treatment was performed on the back skin of the SD rats 3 days in advance. Firstly, 40 mg of imiquimod was applied, and then the ointment was applied at a dose of 1 g/day/rat (application area 5 × 15 cm). The application was performed once a day for 5 consecutive days, while no application was performed in the control group. The skin was wiped to be clean before every application.

**Experimental test:** On day 6, the skin of the rats was wiped to be clean, then the skin was taken to mix and grind with cell lysis solution. After standing, the supernatant was taken to measure the content of IL-17F cell factor in skin.

### Test results:

A represents an IL-17F content less than 2000 pg/g;
B represents an IL-17F content ranging from 2000 pg/g to 5000 pg/g;
C represents an IL-17F content ranging from 5000 pg/g to 10000 pg/g;
D represents an IL-17F content ranging from 15000 pg/g to 20000 pg/g;
E represents an IL-17F content greater than 20000 pg/g.

| Experiment group | Content level of IL-17F in skin |
|---|---|
| Negative control group | A |
| Model control group | E |
| Compound 7 ointment treatment group | A |
| Compound 11 ointment treatment group | A |
| Compound X-1 ointment treatment group | C |

Study on imiquimod induction rat model treated with the ointments of different compounds showed that Compound 7 ointment has better therapeutic effect than Compound X-1 ointment. After the treatment with Compound 7 ointment or Compound 11 ointment by intragastric administration, the level of inflammatory factor IL-17F in the experimental animals was close to that of normal animals. At the same time, it was also observed that after the treatment with Compound 7 ointment or Compound 11 ointment by intragastric administration, the skin lesions in rats were significantly reduced, and the area and severity index score of skin lesions in rats were superior to that of Compound X-1 ointment treatment group. Further experiments show that they are also superior to other compounds with similar skeletons.

### Biological test example 11: Efficacy test in rat model with increased IL-17 induced by imiquimod using application method

Efficacy of the compounds of the present invention was evaluated using the same protocol as biological test example 10, and the following test results were obtained:
A represents an IL-17F content less than 2000 pg/g;
B represents an IL-17F content ranging from 2000 pg/g to 5000 pg/g;
C represents an IL-17F content ranging from 5000 pg/g to 10000 pg/g;
D represents an IL-17F content ranging from 15000 pg/g to 20000 pg/g;
E represents an IL-17F content greater than 20000 pg/g.

| Experiment group | Content level of IL-17F in skin |
|---|---|
| Negative control group | A |
| Model control group | E |
| Compound 2-1A-1 ointment treatment group | A |
| Compound 1-1-1 ointment treatment group | D |
| Compound X-1 ointment treatment group | C |

Study on imiquimod induction rat model treated with the ointments of different compounds showed that Compound 2-1A-1 ointment has better therapeutic effect than Compound 1-1-1 ointment and Compound X-1 ointment. After the treatment with Compound 2-1A-1 ointment by intragastric administration, the level of inflammatory factor IL-17F in the experimental animals was close to that of normal animals. At the same time, it was also observed that after the treatment with Compound 2-1A-1 ointment by intragastric administration, the skin lesions in rats were significantly reduced, and the area and severity index score of skin lesions in rats were superior to that of Compound 1-1-1 ointment and Compound X-1 ointment treatment groups.

### Formulation example 12: preparation of tablet of Compound 2-1A-1

### Composition of the Formulation:

| **Ingredient** | **Function** | **Dosage per tablet (mg)** |
|---|---|---|
| Compound 2-1A-1 | Main ingredient | 12 |
| Starch | Diluent | 30 |
| Microcrystalline cellulose | Diluent | 50 |
| Povidone K30 | Adhesive | appropriate amount |
| Sodium carboxymethyl starch | Disintegrant | 5 |
| Magnesium stearate | Lubricant | 2 |

### Preparation method of the tablet:

**Mixing:** Weighed Compound 2-1A-1, starch, microcrystalline cellulose, and sodium carboxymethyl starch were added into a wet mixing granulator for mixing.

**Preparation of adhesive solution:** Purified water was weighted, slowly added with an appropriate amount of povidone K30 under stirring, and stirred to disperse evenly to prepare the adhesive - an aqueous solution of povidone K30.

**Preparation of soft material:** Wet mixing granulator was used and the stirring speed and shearing speed were controlled, the aqueous solution of povidone K30 was slowly added, stirred and sheared to prepare a mixture material.

**Granulation:** The obtained mixture material was granulated by using an oscillating granulator with 24-mesh sieve to obtain wet granules.

**Drying:** The wet granules were added into a fluidized bed to dry the granules.

**Sieving:** The dried granules were sieved by the oscillating granulator to obtain sieved granules, which were then weighed.

**Mixing:** The sieved granules were added into a three-dimensional multi-directional motion mixer. After mixing, magnesium stearate was added, mixed for about 3 minutes, and subjected to final mixing to obtain the final mixed granules.

**Tablet pressing:** Tablets with complete and neat appearance, uniform color, and suitable hardness and abrasive resistance were prepared by using a tablet press to conduct tablet pressing.

The compounds prepared by the present invention have inhibitory effect on tyrosine kinase 2 (TYK2), thus have a broad application prospect in the treatment of autoimmune diseases and tumors.

The activity and drug-likeness of the compounds of the present invention have prominent characteristics. The compounds with different structural types have different characteristics in absorption, metabolism, and distribution, which are beneficial for providing more effective, convenient, and diverse treatment options for autoimmune diseases and other related diseases in different parts such as enteritis, ankylosing spondylitis, and inflammation involving the skin. In addition, comprehensive positive effects also have been brought in the aspects of preparation of raw materials and formulations, as well as product stability.

## Claims

1. A compound of formula (I): 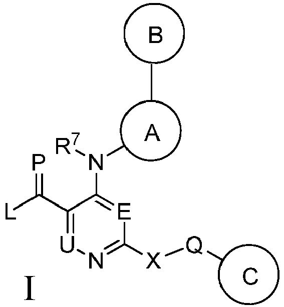 or a stereoisomer, a tautomer, or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
ring B is aryl, heteroaryl, or heterocyclyl, and ring B is selected from: 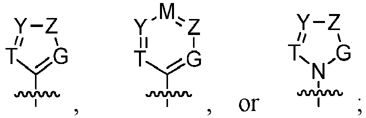
wherein, T, G, Y, Z, and M are each independently selected from oxygen atom, CR^{A1}, CR^{A2}, nitrogen atom or NR^{B};
E is nitrogen atom or carbon atom;
R^{A1} and R^{A2} are selected from the group consisting of hydrogen, deuterium, C₁₋₆ alkyl, halogen, 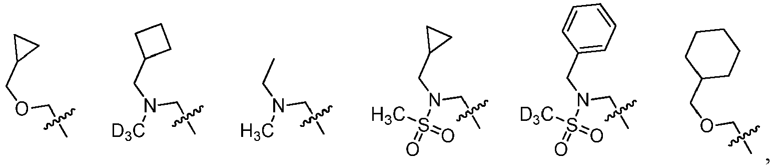 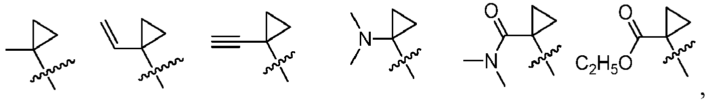 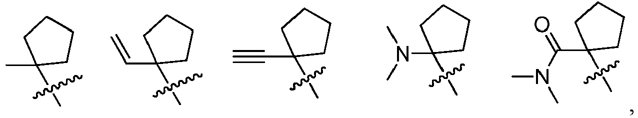 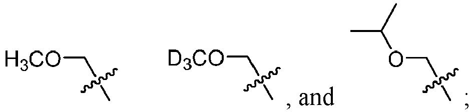
R^{B} is selected from the group consisting of hydrogen, C₁₋₆ alkyl, C₁₋₆ deuterated alkyl, C₁₋₆ alkyl-C(O)-, C₁₋₆ haloalkyl-C(O)-, cycloalkyl-C(O)-, aryl-C(O)-, substituted amino-C(O)-, C₁₋₆ alkyl-S(O)₂-, alkenyl, deuterated alkenyl, alkynyl, deuterated alkynyl, 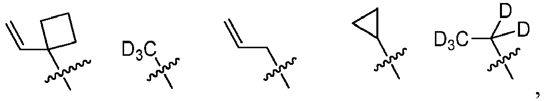 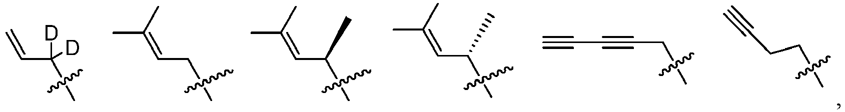 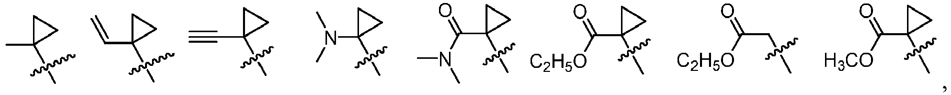 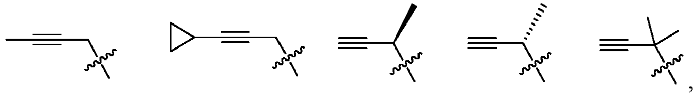 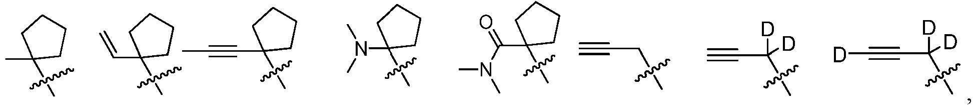 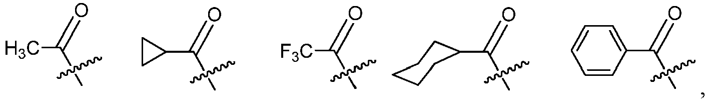 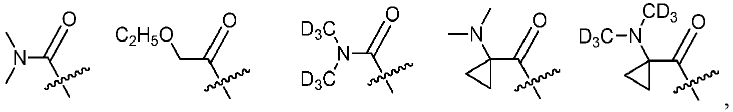 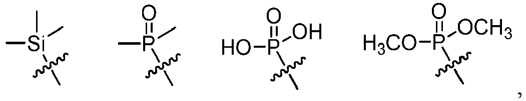 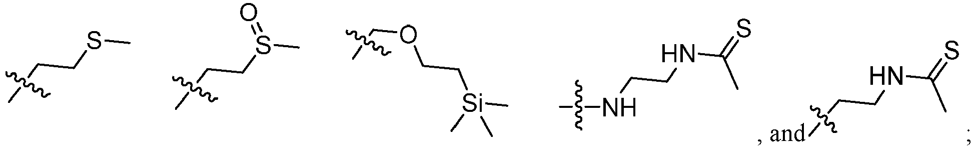
Q is a chemical bond, -C(O)-, -C(S)-, -S(O)-, -S(O)₂-, or -C(N-R⁸)-, wherein -C(O)-, -C(S)-, -S(O)-, -S(O)₂-, and -C(N-R⁸)- are: 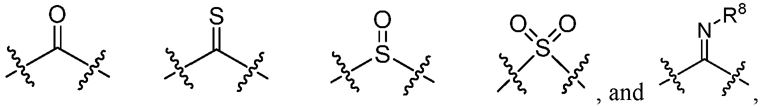 respectively;
P is oxygen atom or sulfur atom;
X is a chemical bond, oxygen atom, NH or NR^{A};
R^{A} is alkyl, deuterated alkyl, or haloalkyl;
U is nitrogen atom or carbon atom; ring A is aryl, heteroaryl, or heterocyclyl, and ring A is selected from the group consisting of: 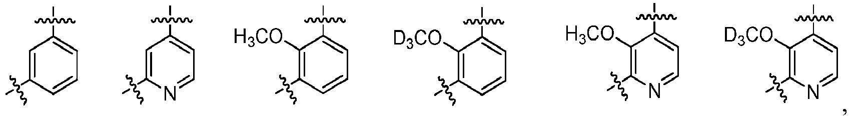 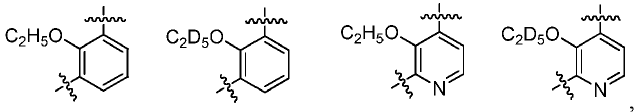 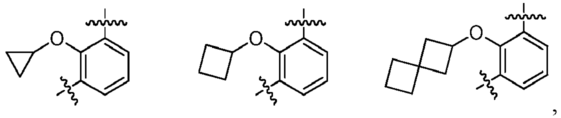 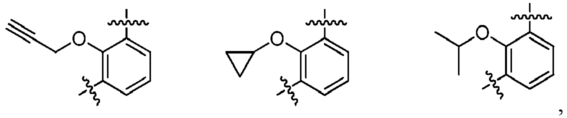 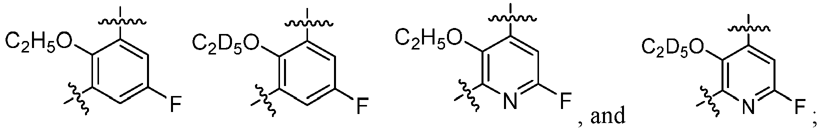
C is alkyl, cycloalkyl, amino, substituted amino, aryl, heteroaryl, or heterocyclyl, and C is selected from the group consisting of: 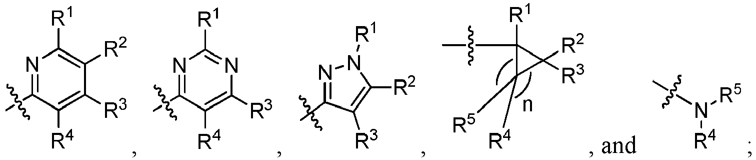
wherein, R¹, R², R³, R⁴, R⁵, R⁷, and R⁸ are selected from the group consisting of hydrogen, deuterium, halogen, amino, alkynyl, deuterated alkynyl, alkenyl, deuterated alkenyl, alkenylcarbonyl, deuterated alkenylcarbonyl, alkyl, deuterated alkyl, alkylcarbonyl, and deuterated alkylcarbonyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
n is 1, 2, 3, 4, 5, or 6.

2. A compound selected from the group consisting of: 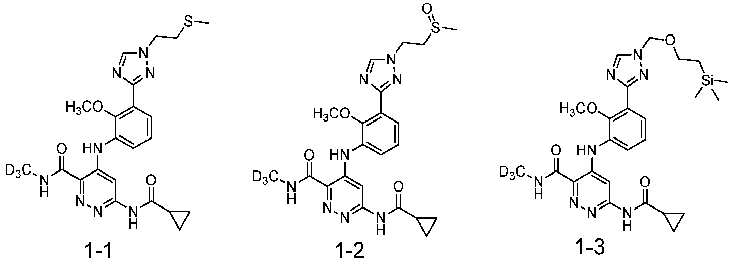 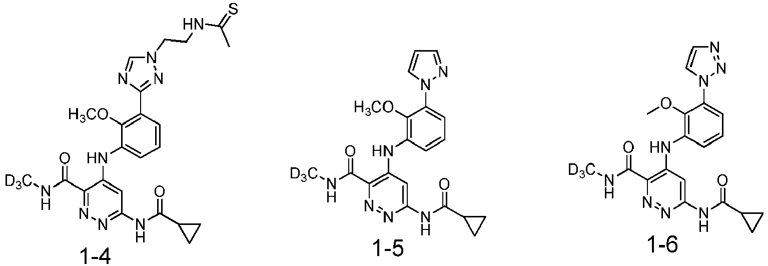 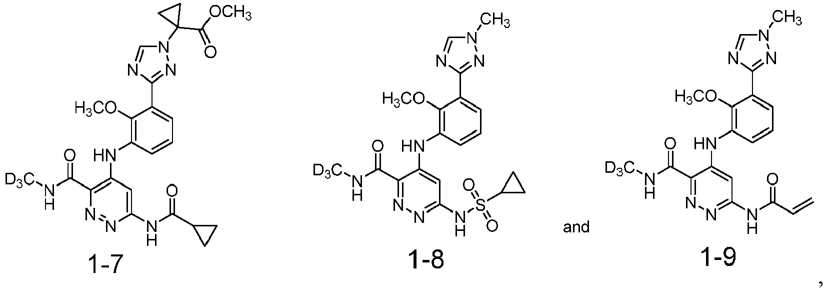 or a stereoisomer, a tautomer, or a pharmaceutically acceptable salt thereof.

3. A compound of formula (I-1): 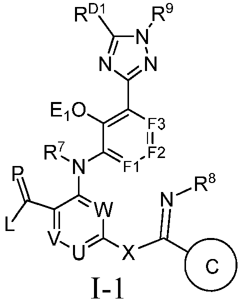 or a stereoisomer, a tautomer, or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
P is oxygen atom or sulfur atom;
X is oxygen atom, NH, or NR⁹;
V, U, and W are nitrogen or CR⁶;
R^{D1} is selected from hydrogen, C₁₋₆ alkyl, or halogen;
F1, F2, and F3 are nitrogen atom or carbon atom;
E₁ is hydrogen, deuterium, alkyl, or deuterated alkyl;
C is alkyl, cycloalkyl, amino, substituted amino, aryl, heteroaryl, or heterocyclyl, and ring C is selected from: 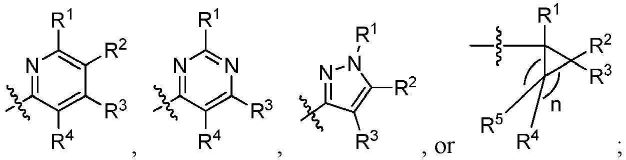
wherein, R¹, R², R³, R⁴, R⁵, R⁶, R⁷, and R⁸ are selected from the group consisting of hydrogen, deuterium, halogen, amino, alkynyl, alkenyl, deuterated alkenyl, alkyl, and deuterated alkyl; wherein the alkynyl, alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

4. A compound selected from the group consisting of: 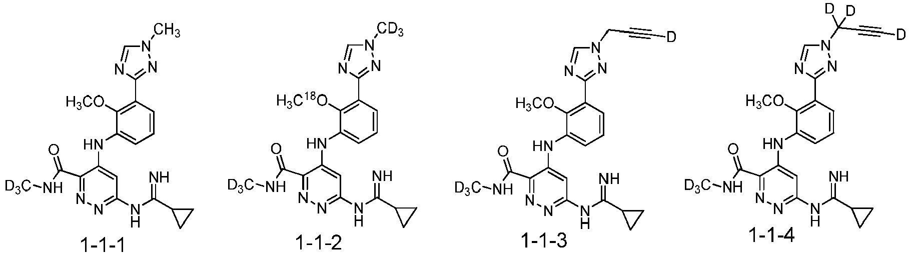 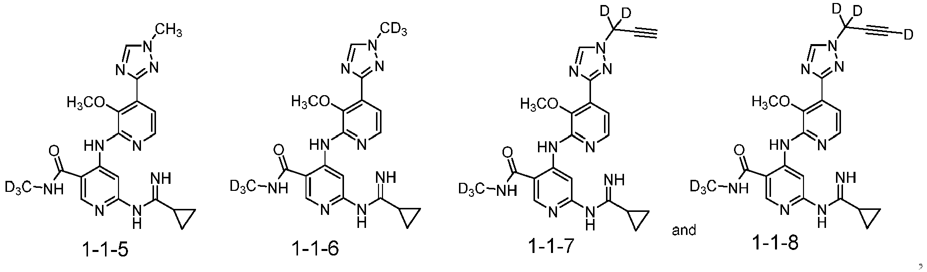 or a stereoisomer, a tautomer, or a pharmaceutically acceptable salt thereof.

5. A compound of formula (II-1): 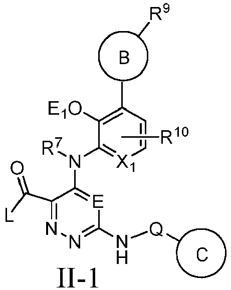 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
Q is a chemical bond, -C(O)-, -C(S)-, -S(O)-, or -S(O)₂-, wherein -C(O)-, -C(S)-, -S(O)-, and -S(O)₂- are: 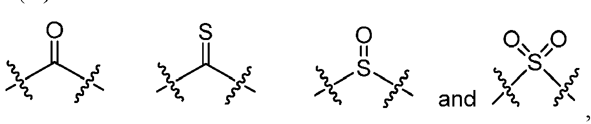 respectively;
C is alkyl, cycloalkyl, amino, substituted amino, aryl, heteroaryl, or heterocyclyl, and C is selected from the group consisting of: 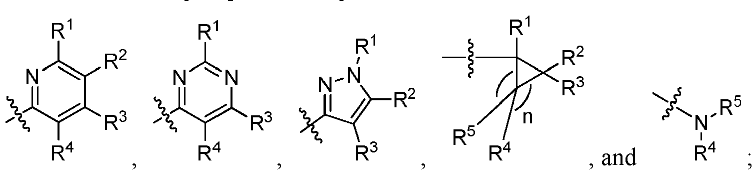
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

6. A compound of formula (II-1A): 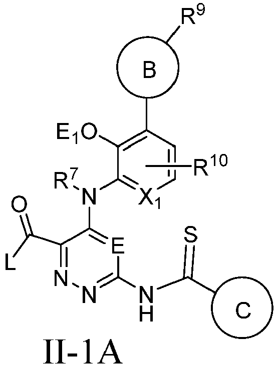 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
C is alkyl, cycloalkyl, amino, substituted amino, aryl, heteroaryl, or heterocyclyl, and C is selected from the group consisting of: 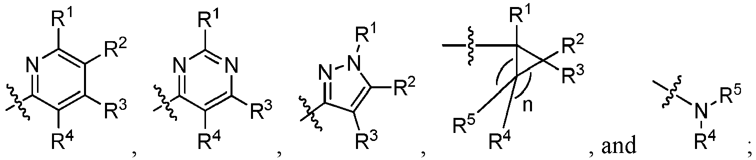
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R⁹ is selected from the group consisting of hydrogen, deuterium, alkyl, deuterated alkyl, haloalkyl, alkoxy, haloalkoxy, halogen, amino, mercapto, nitro, hydroxyl, cyano, oxo, alkenyl, alkynyl, cycloalkyl, heterocyclyl, aryl, heteroaryl, -(CH₂)ₙ₁Rₐₐ, -(CH₂)ₙ₁ORₐₐ, -SRₐₐ, -(CH₂)ₙ₁C(O)Rₐₐ, -(CD₂)ₙ₁Rₐₐ, -(CD₂)ₙ₁ORₐₐ, -SRₐₐ, -(CD₂)ₙ₁C(O)Rₐₐ, -C(O)ORₐₐ, -C(O)Rₐₐ, -S(O)ₘ₁Rₐₐ, -(CH₂)ₙ₁S(O)ₘ₁Rₐₐ, -(CD₂)ₙ₁S(O)ₘ₁Rₐₐ, -NRₐₐR_{bb}, -C(O)NRₐₐR_{bb}, -NRₐₐC(O)R_{bb}, and -NRₐₐS(O)ₘ₁R_{bb};
Rₐₐ and R_{bb} are each independently selected from the group consisting of hydrogen, deuterium, alkyl, deuterated alkyl, haloalkyl, alkoxy, hydroxyalkyl, haloalkoxy, halogen, cyano, nitro, hydroxyl, amino, alkenyl, alkynyl, cycloalkyl, heterocyclyl, aryl, and heteroaryl; wherein the alkyl, deuterated alkyl, haloalkyl, alkoxy, hydroxyalkyl, haloalkoxy, alkenyl, alkynyl, cycloalkyl, heterocyclyl, aryl, and heteroaryl are optionally further substituted with one or more substituents selected from the group consisting of hydrogen, deuterium, silyl, alkylsilyl, substituted or unsubstituted alkyl, halogen, hydroxyl, substituted or unsubstituted amino, oxo, nitro, cyano, substituted or unsubstituted alkenyl, substituted or unsubstituted alkynyl, substituted or unsubstituted alkoxy, substituted or unsubstituted hydroxyalkyl, substituted or unsubstituted cycloalkyl, substituted or unsubstituted heterocyclyl, substituted or unsubstituted aryl, and substituted or unsubstituted heteroaryl;
n is 1, 2, 3, or 4;
n1 is 0, 1, 2, 3, or 4;
m1 is 0, 1, 2, 3, or 4.

7. A compound selected from the group consisting of: 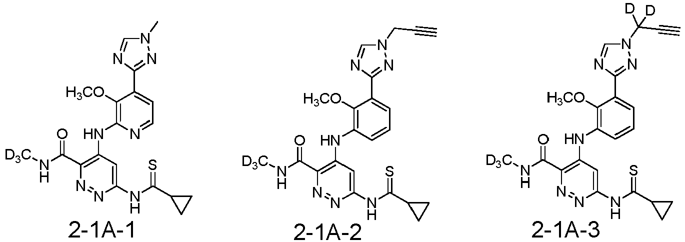 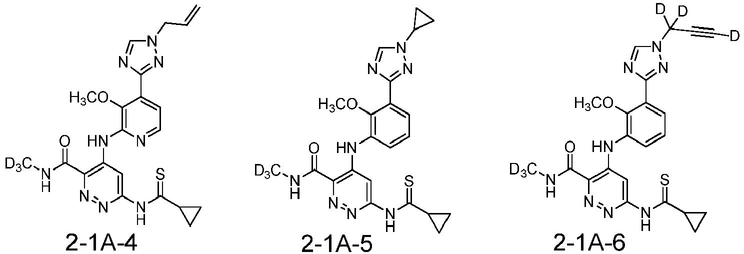 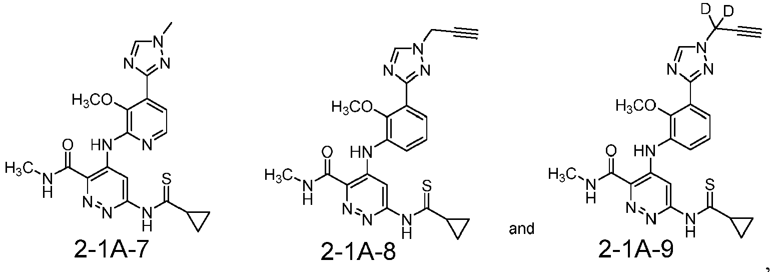 or a stereoisomer, a tautomer, or a pharmaceutically acceptable salt thereof.

8. A compound of formula (II-1B): 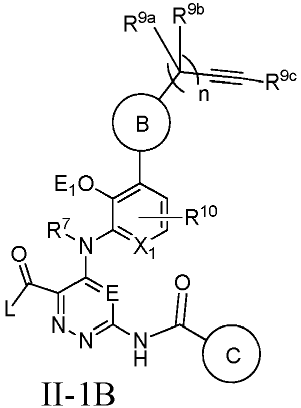 or a stereoisomer, a tautomer, or a pharmaceutically acceptable salt thereof,
wherein, ring B is aryl, heteroaryl, or heterocyclyl;
X₁ and E are nitrogen atom or carbon atom;
L is alkyl, deuterated alkyl, haloalkyl, amino, alkylamino, deuterated alkylamino, cycloalkyl, cycloalkylamino, or deuterated cycloalkylamino;
R¹⁰ is hydrogen, deuterium, alkyl, deuterated alkyl, or halogen;
E₁ is hydrogen, deuterium, alkyl, or deuterated alkyl;
C is alkyl, cycloalkyl, amino, substituted amino, aryl, heteroaryl, or heterocyclyl, and C is selected from the group consisting of: 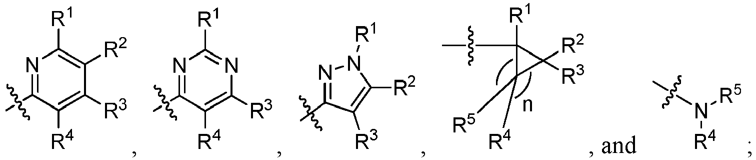
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from the group consisting of hydrogen, deuterium, alkyl, deuterated alkyl, haloalkyl, cycloalkyl, deuterated cycloalkyl, alkynyl, and deuterated alkynyl, or R^{9a} and R^{9b} together with the carbon atom(s) to which they are attached form a cycloalkyl;
n is 1, 2, or 3.

9. A compound selected from the group consisting of: 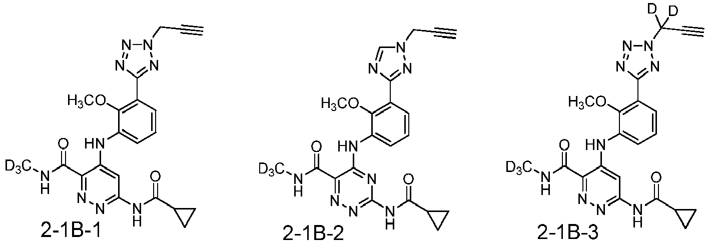 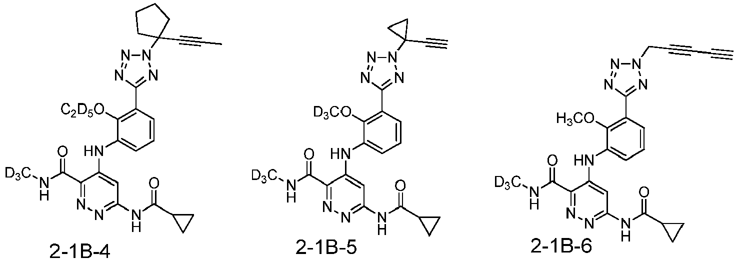 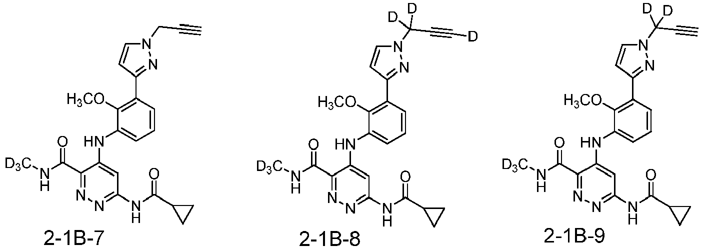 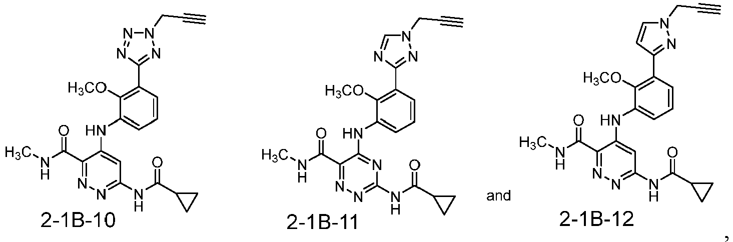 or a stereoisomer, a tautomer, or a pharmaceutically acceptable salt thereof.

10. A compound of formula (II-2): 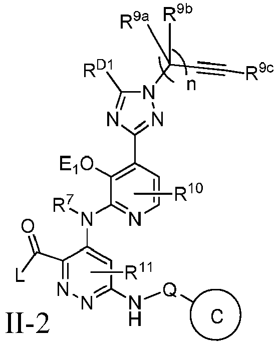 or a stereoisomer, a tautomer, or a pharmaceutically acceptable salt thereof,
wherein, L is alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
Q is a chemical bond or carbonyl;
C is alkyl, cycloalkyl, amino, substituted amino, aryl, heteroaryl, or heterocyclyl, and C is selected from the group consisting of: 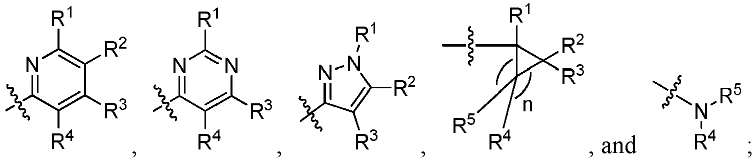
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from the group consisting of hydrogen, deuterium, alkyl, deuterated alkyl, haloalkyl, cycloalkyl, deuterated cycloalkyl, alkynyl, and deuterated alkynyl, or R^{9a} and R^{9b} together with the carbon atom(s) to which they are attached form a cycloalkyl;
n is 1, 2, or 3.

11. A compound of formula (II-2-1): 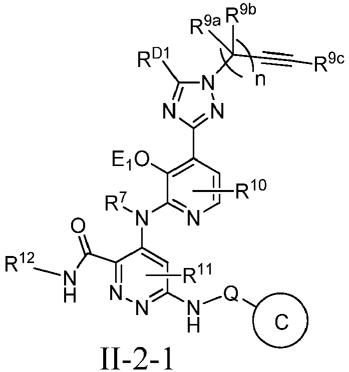 or a stereoisomer, a tautomer, or a pharmaceutically acceptable salt thereof,
wherein, R¹² is deuterium, hydrogen, alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
Q is a chemical bond or carbonyl;
C is alkyl, cycloalkyl, amino, substituted amino, aryl, heteroaryl, or heterocyclyl, and C is selected from the group consisting of: 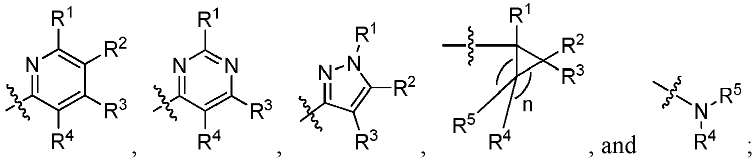
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from hydrogen, deuterium, alkyl, deuterated alkyl, haloalkyl, cycloalkyl, deuterated cycloalkyl, alkynyl, or deuterated alkynyl, or R^{9a} and R^{9b} together with the carbon atom(s) to which they are attached form a cycloalkyl;
n is 1, 2, or 3.

12. A compound of formula (II-2-2): 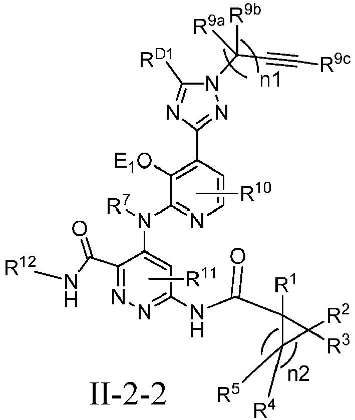 or a stereoisomer, a tautomer, or a pharmaceutically acceptable salt thereof,
wherein, R¹² is deuterium, hydrogen, alkyl, deuterated alkyl, haloalkyl, amino, alkylamino, deuterated alkylamino, cycloalkyl, cycloalkylamino, or deuterated cycloalkylamino;
R¹⁰, R¹¹, and R^{D1} are selected from hydrogen, deuterium, alkyl, deuterated alkyl, or halogen;
E₁ is hydrogen, deuterium, alkyl, or deuterated alkyl;
wherein R¹, R², R³, R⁴, R⁵, and R⁷ are selected from the group consisting of hydrogen, deuterium, halogen, amino, alkynyl, deuterated alkynyl, alkenyl, deuterated alkenyl, alkyl, and deuterated alkyl; wherein the alkynyl, alkenyl, deuterated alkynyl, deuterated alkenyl, alkyl, and deuterated alkyl are optionally substituted with halogen, alkyl, hydroxyl, amino, cycloalkyl, aryl, or heteroaryl;
R^{9a}, R^{9b}, and R^{9c} are selected from hydrogen, deuterium, alkyl, deuterated alkyl, haloalkyl, cycloalkyl, deuterated cycloalkyl, alkynyl, or deuterated alkynyl, or R^{9a} and R^{9b} together with the carbon atom(s) to which they are attached form a cycloalkyl;
n1 is 1, 2, or 3;
n2 is 1, 2, or 3.

13. A compound selected from the group consisting of: 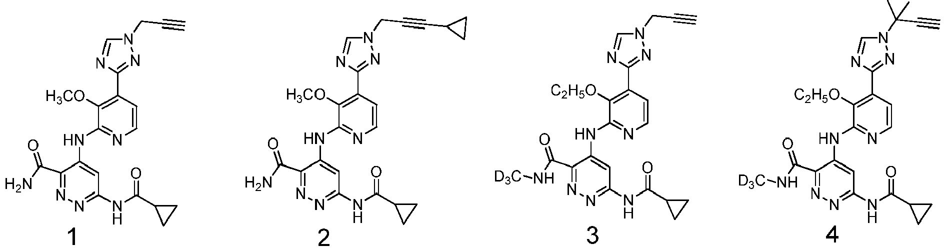 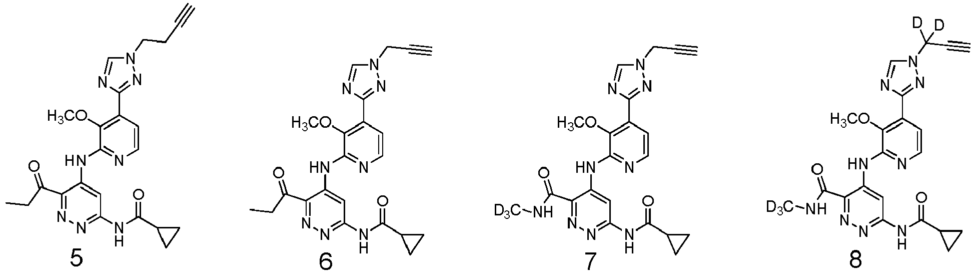 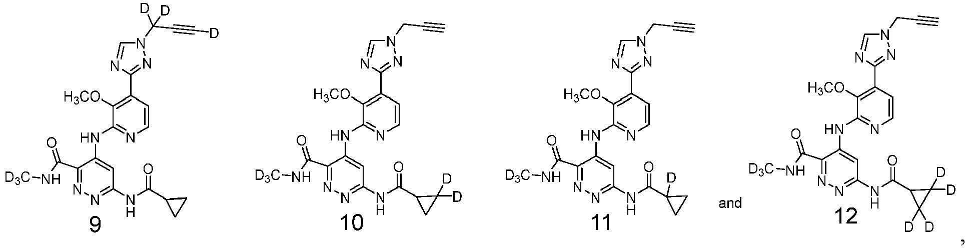 or a stereoisomer, a tautomer, or a pharmaceutically acceptable salt thereof.

14. A pharmaceutical composition, comprising one or more of the compounds according to any one of claims 1 to 13 and a pharmaceutically acceptable carrier or diluent.

15. Use of the compound according to any one of claims 1 to 13 in the manufacture of a medicament for treating a disease, wherein the disease is an inflammatory or autoimmune disease or a cancer mediated by kinase TYK2, including multiple sclerosis, rheumatoid arthritis, inflammatory bowel disease, lupus erythematosus, neurodermatitis, dermatitis, atopic dermatitis, psoriasis, psoriatic arthritis, Crohn's disease, Sjogren's syndrome, or scleroderma.
